(19) Europäisches Patentamt
European Patent Office
Office européen des brevets

(11) EP 4 110 412 B1

(12) EUROPEAN PATENT SPECIFICATION

(45) Date of publication and mention of the grant of the patent:
**26.08.2026 Bulletin 2026/35**

(21) Application number: **21761829.7**

(22) Date of filing: **25.02.2021**

(51) International Patent Classification (IPC):
***A61L 27/18*** (2006.01) ***A61L 27/46*** (2006.01)
***C08G 63/52*** (2006.01) ***C08L 67/04*** (2006.01)

(52) Cooperative Patent Classification (CPC):
(C-Sets available)
**A61L 27/46; C08L 67/04;** A61L 2430/02 (Cont.)

(86) International application number:
**PCT/IN2021/050184**

(87) International publication number:
**WO 2021/171315 (02.09.2021 Gazette 2021/35)**

# (54) A SYNTHETIC COMPOSITE AS BONE GRAFT AND THE METHOD THEREOF

SYNTHETISCHER VERBUNDSTOFF ALS KNOCHENTRANSPLANTAT UND VERFAHREN DAFÜR

COMPOSITE SYNTHÉTIQUE UTILISÉ EN TANT QUE GREFFE OSSEUSE ET SON PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2020 IN 202041008048**

(43) Date of publication of application:
**04.01.2023 Bulletin 2023/01**

(73) Proprietor: **Bone Substitutes**
**Tamil Nadu 625 014 (IN)**

(72) Inventor: **PUGALANTHI PANDIAN, Sankaralingam**
**Madurai, Tamil Nadu 625 014 (IN)**

(74) Representative: **Bandpay & Greuter**
**11 rue Christophe Colomb**
**75008 Paris (FR)**

(56) References cited:
**WO-A1-2015/092814 US-A- 4 888 413**
**US-A- 5 108 755**

- **BOSE SUSMITA, VAHABZADEH SAHAR, BANDYOPADHYAY AMIT: "Bone tissue engineering using 3D printing", MATERIALS TODAY, vol. 16, no. 12, 1 December 2013 (2013-12-01), pages 496 - 504, XP055850032, DOI: https://doi.org/10.1016/j.mattod. 2013.11.01 7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/46, C08L 67/04;**
**C08L 67/04, C08L 67/06, C08K 3/40**

## Description

### FIELD OF INVENTION:

The present invention pertains to the field of composites. Specifically, the invention pertains to the composites of bio-degradable polymers and bioactive fluorophosphate glass as synthetic bone graft, in the form of a powder or a scaffold and the method of making the same.

### BACKGROUND OF THE INVENTION:

The quantity and the type of graft needed by the clinician depends upon the clinical condition and the situation. A surgeon who empties a bone cyst will need a lot of graft to fill the void and in doing so may need a granular filler so that all the nooks and corner is filled up. When the surgeon faces a case on non-union of fracture of a long bone, to bring in bridging callus, apart from good fixation of the fracture he will need to do shingling of the fracture ends to bring out and expose the mesenchymal cells to the exterior and over that lay down strips of iliac bone graft (phemister grafts) to induce and conduct bone union across the fracture site

When a compound fracture with bone loss is encountered, a cortical lesion demands excision of a segment of long bone, then the reconstruction needs a load bearing bone graft which will substitute the lost bone and biologically get converted to bone in a short duration of time without prolonging the morbidity of the patient.

When the metaphyseal end of the bone is diseased and needs removal, without amputating the limb salvage procedures are done with custom made prosthesis. Because the prosthesis has a specific life and can go for fatigue fracture, a custom made graft implant made by rapid prototyping which after implantation will get converted to bone is a boon to the patient than the existing method.

Need of synthetic bone is increasing as the incidence of high speed accidents are increasing and salvage surgeries for bony lesions and tumours are increasing, while the hands of the surgeon are bound by the availability of auto graft because of their limited resource and the morbidity associated with reefing the graft from another site making another one incision. Allograft or bank bone always has the risk of disease transmission.

The material that was put to use initially was ceramics-hydroxyl apatite and tricalcium phosphate as bone graft substitutes. Hydroxyl apatite was only osteo-conductive and rarely was converted to bone even after years. It was not useful in replacing weight bearing function. Tricalcium phosphate had minimal osteo-inductive capacity along with osteo-conduction but had no bio-conversion capability.

To have the advantage of bio-conversion certain specific bone hormones like "Bone Morphogenic Principle" simply called as BMP came into use. Like the same "Demineralised Bone Matrix (DMB)" was also marketed as bone graft substitute. The essential problem in their use is the phenomenal cost involved and they had good osteo-induction but were not good osteo-conductors. HENCH came out with the 45S5 glass which was a breakthrough as it was made from cheap chemicals, were osteo-conductive as well as osteo-inductive, was able to merge with the natural bone and is commercially available. The drawback with 45S5 glass is their very slow resorption, the longer time taken for bio-conversion, and their inability to be used as a weight bearing implant. To circumvent these problems, silica free phosphate bioglass and metal oxide doped bioglass came into the field.

Standardisation of the ideal mole percentage of fluoride resulted in the invention of fluorophosphates glasses which are much more bio active than the phosphate and silica glasses and had a higher rate of bioconversion. Doping them with metal oxides improved their physical properties and brought the elastic moduli close to that of the human bone. Scaffolding the fluorophosphate glass was essential to bring the molecule for clinical use. US4888413 discloses polymer compositions useful for bone repair comprising polymers of fumaric acid and propylene glycol.

Thus, there is a need in the field for a synthetic composite which is biocompatible and has several other characteristics such as being bioactive, biodegradable; nontoxic to the recipient; bio conductive; undergoes bioconversion, workable to the desired shape and cost effective

### OBJECT OF INVENTION:

The object of the invention is for synthetic composites of bio inert polymers comprising of poly lactic acid, poly D, L -Lactic acid and bio active polymers consisting of polypropylene fumarate, diester of fumaric acid and propylene diol (1,2 Diol) and a bioactive inorganic component consisting of a metal fluorophosphates glass powder.

Another object of the invention is for granules, scaffolds like strips, cylinders and any other shape of the composites and the method of making the same.

Another object of the invention is for a scaffold preparation by 3 D printing.

## SUMMARY OF INVENTION:

The invention is for a synthetic composite for a bone graft comprising of: bio inert polymers comprising poly lactic acid, poly D, L -Lactic acid; bio active polymer consisting of polypropylene fumarate or diester of fumaric acid and propylene diol (1,2-Diol); and a bioactive inorganic component consisting of a metal fluorophosphates glass powder wherein the amount of the bioactive components is upto 30% (w/w) of the composite.

In an aspect of the invention the bioactive inorganic metal fluorophosphates glass powder of the composite is one of zinc fluorophosphate, magnesium fluorophosphate or silver fluorophosphate.

In an aspect, the polylactic acid in the composite is in the range of 54% (w/w) to 68% (w/w); poly D, L -lactic acid is in the range of 10% (w/w) to 28% (w/w); 1,2 diol is in the range of 3% (w/w) to 10% (w/w); polypropylene fumarate is in the range of 3% (w/w) to 10% (w/w); the inorganic metal fluorophosphates glass powder in the composite is in the range of 10% (w/w) to 30% (w/w).

In an embodiment the composite is in the form of a powder, or a scaffold. The scaffold is a strip or a cylinder or a tube and the like as and when fabricated.

In an embodiment the synthetic composite of the invention is prepared by the following method comprising the steps of: mixing the composite in a solvent with the magnetic stirrer or sonicated to obtain a homogenous mixture; the mixture is casted over hot glass plate and the solution is brought to boil; and evaporation of the solvent by continuous boiling; and an interconnected porous scaffold with the homogenous distribution of the components of the composite was obtained.

The porosity of the scaffold ranges from 20%-40%.

In addition, the scaffold is also made of desired shape and desired porosity by a custom-made 3D printer by the Direct Ink Printing Technique.

The method of obtaining a scaffold by a custom-made 3D printer by the Direct Ink Printing Technique comprising the following steps: The components of the composites are homogenised and cooled to 10-30°C. The chamber temperature is kept at 30-40°C. The ink is loaded into a pressure-controlled, non adherent extruder. The input writing details were fed to the printer through the microchip. The nozzle diameter was fixed as 300μm for easy extrusion. The volumetric flow rate was set at $5mm^3/s$. The space between the lines of writing was 200 microns and the number of layers are designed 10. The desired shape, thickness, porosity, layers fed by a computer is written on a hot plate (100°C). The movement of the hot plate in x,y,z direction were also pre-set and the commands was transferred by the microchip .

## BRIEF DESCRIPTION OF DRAWINGS AND FIGURES:

Figure 1 is bar diagram depicting the ALP activity of the dissolution products of metal oxide doped FP glasses at various concentrtions of the glass.
Figure 2a and 2b. Thermal studies of the FP and MgFP glass powder.
Figure 2c and 2d. Thermal studies of the ZnFP and TiFPglass powder.
Figure 2e and 2f. Thermal studies of the ZrFPand AgFPglass powder.
Figure 2g. Thermal studies of the SrFPglass powder.
Figure 3 represents the FTIR spectrum of PPF.
Figure 4. represents the DSC study of the PPF and figure 4a. represent the transition glass temperature of the PPF.
Figure 5. FTIR spectrum of the Fumaric acid 1,2 propane diol.
Figure 6. TG study of the Fumaric acid 1,2 propane diol.
Figure 7. Characterization and Thermal analysis of the PLA.
Figure 8. Characterization and Thermal analysis of the PDLLA.

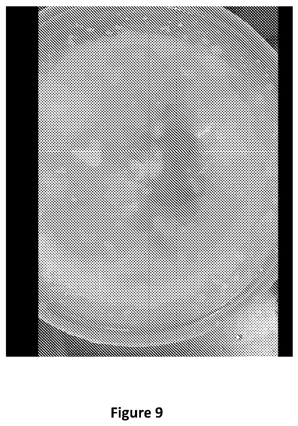

Figure 9. Preparation of flurophosphate glass by quenching from 1200 degrees to instant-170° C.
Figure 10. Attempts of scaffolding by varying the concentration of polymers.
Figure 11. Scaffolding attempts by varying the concentration of FP salts.
Figure 12. Cell adhesion studies of the scaffold with relation to the variation in the components.
Figure 13. Cell adhesion studies of the composites in relation to the variation in the components and the presence or absence of porosity.
Figure 14. Different scaffolds achived by different methods of scaffolding.
Figure 14a. SEM image of the scaffold made by Gel compression.
Figure 14b. Preparation of scaffold by gel foam casting under rapid heating .
Figure 15. Cytotoxicity (MTT) Assay of Endothelial Cells of scaffolds.
Figure 15a. Cytotoxicity (MTT) Assay on Endothelial Cells of scaffolds (photomicrograph).
Figure 16. RT-PCR Collagen II expression of the AgFP and ZnFP based scaffolds.

Figure 17. RT-PCR Osteocalcin expression of the AgFP and ZnFP based scaffolds.
Figure 18. RT-PCR Collagen II and Osteocalcin expression of Mg based scaffolds.
Figure 19. RT_PCR RUN_X2 expression of scaffolds.
Figure 20. Chondroitin sulphate levels of scaffolds' expression in SaOS2 cell lines.
Figure 21. FTIR Spectra of the PPF based scaffolds (In-vitro evaluation -pre and post immersion).
Figure 21a.Interpretation of the PPF based scaffolds (In-vitro evaluation -pre and post immersion).
Figure 22. FTIR Spectra of the Diol based scaffolds (In-vitro evaluation- pre and post immersion).
Figure 22a.Interpretaion of the Diol based scaffolds (In-vitro evaluation - pre and post immersion).
Figure 23. FTIR Spectra of the MgFP based scaffolds (In-vitro evaluation- pre and post immersion).
Figure 23a.Interpretaion of the MgFP based scaffolds (In-vitro evaluation - pre and post immersion).
Figure 24. FTIR Spectra of the scaffolds of AgFP, ZnFP, MgFP (scaffolded by gel foam casting under rapid heating) (In-vitro evaluation -pre and post immersion).
Figure 24a.Interpreation of the scaffolds of AgFP, ZnFP, MgFP (scaffolded by gel foam casting under rapid heating) (In-vitro evaluation -pre and post immersion).
Figure 25. FTIR Spectra of the strip scaffold (in vitro evaluation- Pre and Post immersion).
Figure 25a.Interpretaion of the strip scaffold (in vitro evaluation- Pre and Post immersion).
Figure 26. FTIR Spectra of the cylindrical scaffold (in-vitro evaluation-Pre and Post immersion).
Figure 26a.Interpretation of the cylindrical scaffold (in vitro evaluation-Pre and Post immersion).
Figure 27. Photograph of strip and cylindrical scaffold made by gel foam casting under rapid heating.
Figure 28. SEM micrograph of the Pre and Post immersion scaffold in two different magnification.
Figure 28a. Depth of crystallisation (from both superior and inferior surface) -inner zone of the scaffold evaluated by SEM.
Figure 29. Micro CT evaluation of the pre in-vitro of the cylindrical sample
Figure 29a. Micro CT evaluation of the post in-vitro of the cylindrical sample
Figure 30. SEM images of the cylindrical scaffold (pre immersion)
Figure 30a. SEM images of the cylindrical scaffold (post immersion)
Figure 30b. EDAX of the specimens pre and post in vitro evaluation.
Figure 31 SEM image of a strip of scaffold (pre immersion and post immersion).
Figure 32. Animal study to assess the efficacy of the granules of the scaffold.
Figure 33. Post-operative X-ray of the femur bone.
Figure 34. X-Ray of the Dissected specimen.
Figure 35. Segment of the specimen studied in the HPE.
Figure 36. Histo pathological evaluation of the specimen (EH stain and von kossa stain)
Figure 37-a-b-c-d. Modified Tetrachrome staining of the specimen
Figure 38. Animal study to assess the efficacy of Strips of the composites.
Figure 39,39a,39b. Day 0 & Day1, Day 9, Day 15 x-rays of the three animals (A,B,C AgFP, ZnFP, MgFP respectively).
Figure 40, 40a.40b. CT.scan on day 19 of all three animals. (AgFP, ZnFP, MgFP respectively).
Figure 41. Photographs of the dissected specimens (AgFP, ZnFP, MgFP respectively).
Figure 42. X-Ray of the dissected specimens (AgFP, ZnFP, MgFP respectively).
Figure 43 a,b,c,d. Histo pathological evaluation of the specimens(EH and Masson Trichrome stain).
Figure 44 a,b,c,d. Histo pathological evaluation of the specimens by Modified Tetrachrome stain.
Figure 45 (a) The control panel of the designed 3D printer.
Figure 45(b) The pressure controlled, temp controlled extruder and the temp controlled table top.
Figure 45 (c) The printer in the process of printing and the printed specimens.

**Table legends**

Table 1. Extracellular osteocalcin secretion by ionic dissolution products of various metal oxide doped FP glass in MG 63 cells.
Table 2. Intracellular osteocalcin secretion by ionic dissolution products of various metal oxide doped FP glass in MG 63 cells.
Table 3. Preparation of scaffold by varying the proportion of FP glass.
Table 4.Effect of cell adherence over the scaffold (as in Table 4) in MG 63 cell lines.
Table 5.Preparation of scaffold by increasing the percentage of bioactive components PPF and FP GLASS at the cost of reducing the bioinert component PLA and PDLLA.
Table 6. Effect of cell adherence over the scaffold (as in Table 5) in MG 63 cell lines.

Table 7. Preparation of scaffold by varying the percentage of the Bioinert components PLA and PDLLA keeping the Bioactive components fixed(PPF and Glass powder).
Table 7a. Preparation of scaffold by varying the percentage of the Bioinert components PLA and PDLLA keeping the Bioactive components fixed(1,2-Diol and Glass powder).
Table 8. Effect of cell adherence over the scaffold (as in Table 7) in MG 63 cell lines.
Table 8a. Effect of cell adherence over the scaffold (as in Table 7a) in MG 63 cell lines.
Table 9.MTT of SaOS2 cell line varying according to the variation in the components and to the presence of pores.
Table 10a and 10b: Composites and the proportion of the componets in the composite.
Table 11. MTT of the 12 types scaffolds (varying components, +/- PPF/XPPF, +/- Pores) in SaOS 2 Cell line.
Table 12. ALP activity of composites.
Table 13. Chondroitin levels of scaffolds.
Table 14.Comparison chart of the biological activity of scaffolds.
Table 15. Invitro studies- pH variation of the simulated body fluid (SBF) of scaffolds over 21 days.
Table 16.XRD results of the compression moulded scaffold after SBF immersion.
Table 17.XRD results of the rapid heating scaffolds after SBF immersion.
Table 18. Percentage of crystallisation of scaffold in the in-vitro study.

## DETAILED DESCRIPTION:

The invention is for a synthetic composite for a bone graft comprising of: bio inert polymers comprising poly lactic acid, poly D, L -Lactic acid; bio active polymer consisting of polypropylene fumarate or diester of fumaric acid and propylene diol (1,2-Diol); and a bioactive inorganic component consisting of a metal fluorophosphates glass powder wherein the amount of the bioactive components is upto 30% (w/w) of the composite.

The bioactive inorganic metal fluorophosphates glass powder of the composite is one of zinc fluorophosphate, magnesium fluorophosphate or silver fluorophosphate.

The polylactic acid in the composite is in the range of 54% (w/w) to 68% (w/w); poly D, L - lactic acid is in the range of 10% (w/w) to 28% (w/w); 1,2 diol is in the range of 3% (w/w) to 10% (w/w); polypropylene fumarate is in the range of 3% (w/w) to 10% (w/w); the inorganic metal fluorophosphates glass powder in the composite is in the range of 10% (w/w) to 30% (w/w).

In an aspect the composite comprises of polylactic acid, 1,2 diol, and zinc fluorophosphate.

In an aspect the composite comprises of polylactic acid, poly D, L-Lactic acid, 1,2 diol and zinc fluorophosphate.

In an aspect the composite comprises of polylactic acid, poly propylene fumarate and zinc fluorophosphate.

In an aspect the composite comprises of polylactic acid, poly D, L-Lactic acid, poly propylene fumarate and zinc fluorophosphate.

In an aspect the composite comprises of polylactic acid, 1, 2 diol and magnesium fluorophosphate.

In an aspect the composite comprises of polylactic acid, poly D, L -Lactic acid, 1,2 diol and magnesium fluorophosphate.

In an aspect the composite comprises of polylactic acid, poly propoylene fumarate and magnesium fluorophosphate.

In an aspect the composite comprises of polylactic acid, poly D, L, lactic acid, poly propylene fumarate and magnesium fluorophosphate.

In an aspect the composite comprises of polylactic acid, 1, 2 diol and silver fluorophosphate.

In an aspect the composite comprises of polylactic acid, poly D, L -Lactic acid, 1,2 diol and silver fluorophosphate.

In an aspect the composite comprises of polylactic acid, poly propylene fumarate and silver fluorophosphates.

In an aspect the composite comprises of polylactic acid, poly D,L, lactic acid, poly propylene fumarate and silver fluorophosphate.

The composite is in the form of a powder, or a scaffold. The scaffold is a strip or a cylinder or a tube and the like as and when fabricated.

The synthetic composite of the invention is prepared by the following method comprising the steps of: mixing the composite in a solvent with the magnetic stirrer or sonicated to obtain a homogenous mixture; the mixture is casted over hot glass plate and the solution is brought to boil; and evaporation of the solvent by continuous boiling; and an interconnected porous scaffold with the homogenous distribution of the components of the composite was obtained.

In an aspect the solvent used in the method is one of dichloromethane, acetone, or toluene, or chloroform.

The porosity of the scaffold ranges from 20%-40%.

In addition, the scaffold is also made of desired shape and desired porosity by a custom-made 3D printer by the Direct Ink Printing Technique.

The method of obtaining a scaffold by a custom-made 3D printer by the Direct Ink Printing Technique comprising the following steps: The components of the composites are homogenised and cooled to 10-30°C. The chamber

temperature is kept at 30-40°C. The ink is loaded into a pressure-controlled, non adherent extruder. The input writing details were fed to the printer through the microchip. The nozzle diameter was fixed as 300μm for easy extrusion. The volumetric flow rate was set at $5mm^3/s$. The space between the lines of writing was 200 microns and the number of layers are designed 10. The desired shape, thickness, porosity, layers fed by a computer is written on a hot plate (100°C). The movement of the hot plate in x,y,z direction were also pre-set and the commands was transferred by the microchip.

In an aspect the biological evaluation of the fluorophosphate glass was ascertained by their MTT, their intracellular and extracellular osteocalcin secretion and also ALP secretion in relation to MG63 cell lines.

In an aspect the significance of the pores in the scaffold was assessed by calceinAM study and MTT evaluation.

In an embodiment the biological potential of the different composites with different composition of the components have been ascertained by the MTT of the composites in relation to the SaOS2 and Human Endothelial cell lines, their efficiency in enhancing secretion of Alkaline phosphatase, Chondroitin sulphate the ground substance in the bone.

In addition, the ability of the composites in the secretion of osteocalcin, collagen II, RUN_X2 were assessed by RT-PCR method. The porosity in the multi-layered scaffold was assessed by MicroCT evaluation.

In an embodiment, *invitro* study of the various composites and the various scaffolds were done by immersing in SBF for 21 days and were then studied by their XRD, FTIR, SEM, and MicroCT.

In an embodiment the bone forming efficacy of the composite was assessed by *in-vivo* evaluation in rabbits, confirmed by histopathological evaluation.

In an aspect the synthetic composite has following characteristics

a) Biocompatible; b) Bioactive; c) Biodegradable; d) Nontoxic to the recipient;e) Bioconductive; f) Bioinductive; g) Bioconvertible; h) Rate of degradation to match the rate of bio-conversion; i) sterilisable; j) easy to be produced in bulk; k) workable to the desired shape; l) cost effective

**EXAMPLES:**

The following examples are for the purpose of illustration of the invention and are not intended in any way to limit the scope of the invention.

**MATERIALS:**

Poly lactic acid (PLA) and poly DL-lactic acid (PDLLA) were procured from BioDegmer® Japan. Polypropylene fumarate (PPF) and diester of fumaric acid and propylene diol (1,2 Diol) was procured from Department of Polymer Technology, Kamaraj College of Engineering and Technology, S.P.G.C. Nagar, K.Vellakulam-625 701, India.

The polymers have been synthesized at the Department of Polymer Technology, Kamaraj College of Engineering and Technology. The method involves addition of diethyl fumarate, 1,2 propane diol, zinc chloride (catalyst) and hydroquinone (crosslinking inhibitor) in reaction vessel in the molar ratio of 1.0:3.0:0.01:0.002. The reaction vessel was fitted with double walled condenser and the receiving flask connected to it for by product collection. The system was kept in an oil bath at 100 °C with efficient magnetic stirring with subsequent application of vacuum (-80mmHg). The temperature was raised to 150°C with constant stirring, esterification condensation reaction occurred. As a result of this, the intermediate bis (hydroxypropyl) fumarate diester was formed and ethanol was distilled as the primary by product. After the expected amount of ethanol collection, transesterification reaction was carried out with the elimination of excess amount of 1,2-propane diol as secondary by product. Now, the synthesized material was dissolved in acetone. This solution was repeatedly washed with ice cold distilled water to remove the unreacted reactants and catalyst. A sufficient amount of anhydrous sodium sulphate was added to the acetone solution of the polyester so as to dry the acetone solution. After filtration, the solvent was slowly evaporated in hot air oven at 50 °C to yield PPF.

Fumaric acid (1.0 mol) and 1,2-propane diol (2.2 mol) were taken in round bottom flask and p-toluene sulphonic acid was used as the esterification catalyst. The dry toluene was added to the reaction mixture in order to remove the water formed during the esterification as an azeotrope. The Dean Stark apparatus was used for the above purpose. The reaction vessel was submerged in an oil bath, mixed uniformly and continuously using magnetic stirrer. The temperature was initially set at 100 °C and gradually raised to 140 °C in 10 °C increments over a period of one hour. When the temperature reached 110 °C (boiling point of toluene), water starts to collect in the Dean Stark apparatus. The reaction was allowed to proceed till the stipulated quantity of water was collected. The material was purified by applying vacuum to remove the excess unreacted 1,2-propane diol and water.

The FP glass component of the invention was procured from Bone Substitutes, Madurai. The method of preparation is as outlined in Indian patent application 5760/CHE/2013, 5990/CHE/2013, 5989/CHE/2013 and cited as references for the preparation of FP glasses. The method is briefly outlined below. The measured quantities of the required chemicals ($Na_2CO_3$, $CaCO_3$, $CaF_2$, $P_2O_5$ and $ZnO/Ag_2O/MgO$) were taken in a ball mill and homogenised. The mixture was heated in the alumina crucible for 1h upto 120°C and cooled to room temperature. It was again ball milled for 1hr.The components were taken in a platinum crucible and kept in a furnace preheated to 1100°C and allowed for 90mts.Then the

crucible was sunk into a bowl having liquid nitrogen. The formed glass was broken to pieces and milled for 48h to obtain nano powder of the specific fluorophosphates glass. The FP glass material was prepared at Bone Substitutes, Madurai, India.

The MG-63 (ATCC® CRL-1427™) were obtained from The National Centre for Cell Science (NCCS), Pune, INDIA

The Saos-2 (ATCC® HTB-85™) was obtained from The National Centre for Cell Science (NCCS),Pune, INDIA

**EXAMPLE 1: Selection of non toxic inorganic metal fluorophosphates glass powder.**

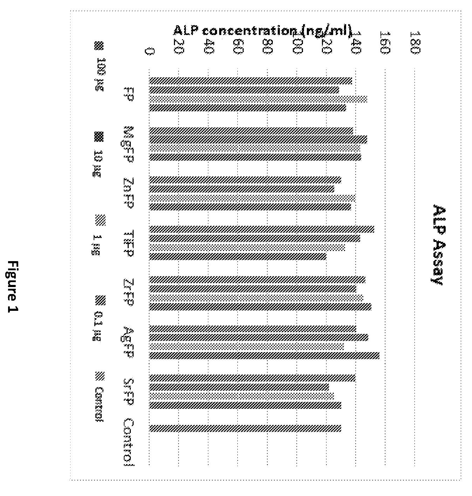

a)MTT Proliferation Assay: The MG-63 cells were cultured into 24 well plates and ionic dissolution products of metal doped bio glass (fluorophosphate (FP), Magnesium fluorophosphate ( MgFP), Zinc fluorophosphate (ZnFP), Titanium fluorophosphate (TiFP), Zirconium fluorophosphate (ZrFP), Silver fluorophosphate (AgFP) and strontium fluorophosphate (SrFP)) were co treated with cells on 0 hr seeding and monitored till 48 h to study cell morphology and after that the cells were washed twice with 1XPBS before being incubated with 0.2 mg/mL of MTT (3 -(4,5-dimethylthaizole-2-yl)-2,5-diphenyl tetrazolium bromide) for 2 h. The purple colored product formed was then dissolved with isopropyl alcohol and the optical density was measured at 570nm using ELISA Reader (Robonik, India) (Figure 1). Fig 1 shows all the metal oxide doped Fluorophosphate glasses were nontoxic and their viability exceeded 80% after 48 hrs of incubation (up to 10microgram per ml.)

Alkaline phosphatase (ALP) is an essential enzyme in the process of bone formation from the mesenchymal cells to the mineralisation front. Hence its enhanced secretion is considered a vital factor to choose the ingredient for the composite for Bone Tissue Engineering (BTE). The results of the study in ALP secretion shows AgFP, ZrFP and MgFP showed consistently raised levels at all concentration from 0.1-100 μg/mL. ZnFP showed increased secretion only at lower concentrations of 0.01 and 1 μg/mL (Figure 1).

b) Thermal evaluation:

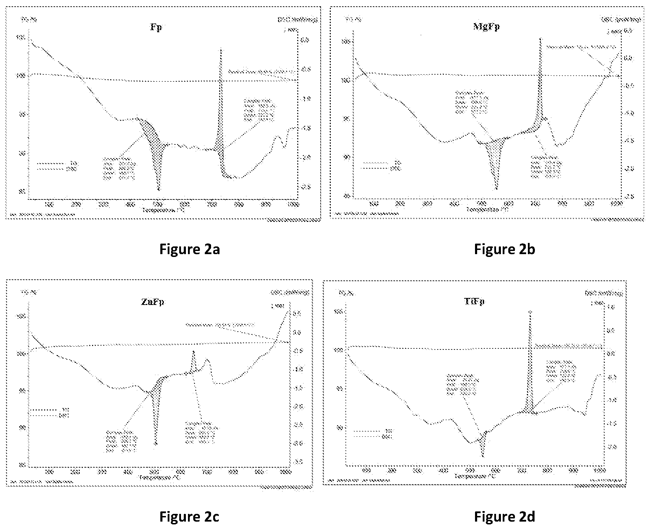

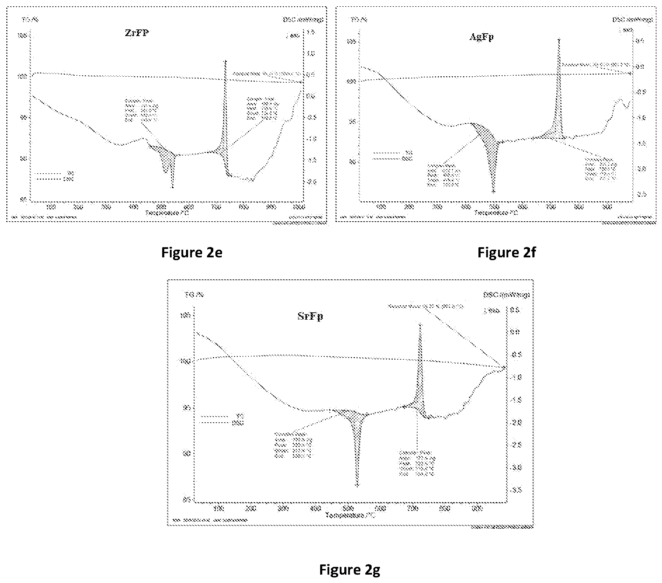

The Simultaneous Thermal Analysis (STA 449 F3Nevio) was used to obtain the thermal stability of the bioglass. 3.5mg of metal fluorophosphates (each) was heated till 1000°C at 50K $min^{-1}$ in nitrogen atmosphere. (Figure 2a-2g).

The thermal evaluation of all the seven types of FP Glasses revealed that their Tg (glass transition temperature) was between 500-550°C and their Tc (crystallisation temperature) was around 700°C indicating a large window width of around 150°C which can be useful in sintering while scaffolding (Figure 2a-2g).

c) Osteocalcin (intracellular and extracellular) assay by ELISA method:

Osteocalcin secreted by MG-63 in response to the addition of ionic dissolution products of each fluorophosphates bio glass samples in 100 μg, 10 μg and 1 μg concentrations into the culture (extracellular as wells as intracellular) and responses were analysed by ELISA.

MG-63 cells were seeded into 24 well plates (2 X $10^5$ cells/well). After overnight adherence, media was removed and washed with Dulbecco's PBS. Ionic dissolution products of various Fluorophosphate bioglass samples with various concentrations were added to the wells (media without phenol red, serum and antibiotic). The assay plates were kept in $CO_2$ incubator with 5% $CO_2$ at 37°C for 72 h. After incubation, supernatants were taken for the analysis of osteocalcin expression in extracellular environment.

For the assessment of intracellular expression of osteocalcin, cells from the wells were detached using Accutase (Gibco) and collected. 200 μL of cell lytic solution (Sigma) was added to each well and incubated for 10 m. Lysed cellular components were centrifuged and supernatant was taken for intracellular assessment. 100 μL from each sample was taken for evaluation by the ELISA method. Experiment was performed according to the instructions provided by the manufacturer (DIA source hOST-EASIA Kit, Belgium). Absorbencies were read at 450 nm. The expression of osteocalcin was calculated by plotting standard curve and values were expressed in ng/mL(Table 1 &2).

Table 1 Extracellular osteocalcin secretion by ionic dissolution products of various metal oxide doped FP glass in MG 63 cells

| | | **Extracellular (concentration in ng/mL)** | | | |
|---|---|---|---|---|---|
| **S. No** | **Sample** | **100 μg** | **10 μg** | **1 μg** | **Control** |
| 1 | FP | 0.179 | 0.492 | 0.247 | 0.430 |
| 2 | Mg FP | 0.317 | 0.300 | 0.469 | 0.430 |

(continued)

| | | Extracellular (concentration in ng/mL) | | | |
|---|---|---|---|---|---|
| **S. No** | **Sample** | **100 μg** | **10 μg** | **1 μg** | **Control** |
| 3 | Zn FP | 0.324 | 0.557 | 0.208 | 0.430 |
| 4 | TiFP | 0.392 | 0.323 | 0.326 | 0.430 |
| 5 | ZrFP | 0.257 | 0.421 | 0.271 | 0.430 |
| 6 | Ag FP | 0.274 | 0.194 | 0.319 | 0.430 |
| 7 | SrFP | 0.306 | 0.302 | 0.220 | 0.430 |

Table 2 showing extracellular expression of osteocalcin in ng/mL

| | | Intracellular (concentration in ng/mL) | | | |
|---|---|---|---|---|---|
| **S. No** | **Sample** | **100 μg** | **10 μg** | **1 μg** | **Control** |
| 1 | FP | 0.009 | 0.100 | 0.039 | 0.083 |
| 2 | Mg FP | 0.207 | 0.084 | 0.091 | 0.083 |
| 3 | Zn FP | 0.000 | 0.251 | 0.011 | 0.083 |
| 4 | TiFP | 0.083 | 0.000 | 0.288 | 0.083 |
| 5 | ZrFP | 0.003 | 0.099 | 0.005 | 0.083 |
| 6 | Ag FP | 0.253 | 0.028 | 0.096 | 0.083 |
| 7 | SrFP | 0.033 | 0.246 | 0.049 | 0.083 |

Bone is a composite of the ground substance reinforced by multiple collagens and mineralised by hydroxyl apatite. Though various collagens are present in various parts of the body osteocalcin is found exclusively in bone. It is also an excellent gene marker of bone induction. The ability of the ionic dissolution products of various FP glasses in various concentration were evaluated for their efficiency to promote osteocalcin secretion. While the extra cellular expression of osteocalcin showed increase than the control only with ZnFP and MgFP, (Table 1) intracellular osteocalcin was raised in most of the glasses but significant raise was present in ZnFP, MgFP and AgFP glasses and was more when the concentration of the products of dissolution was 10 μg/mL(Table2).

Based on the above studies and the characteristics of the fluorophosphate glasses AgFP, ZnFP, MgFP were selected for the composite preparation and for the fabrication of a composite into desired structure and shape.

**EXAMPLE 2:**

a) Selection of Biopolymers and characterization

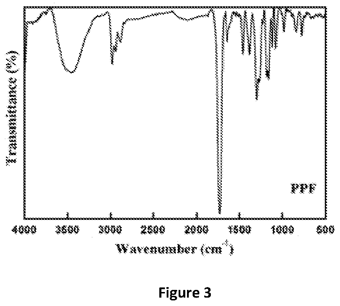

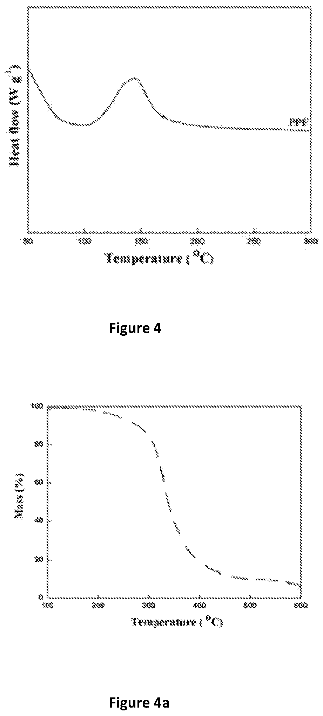

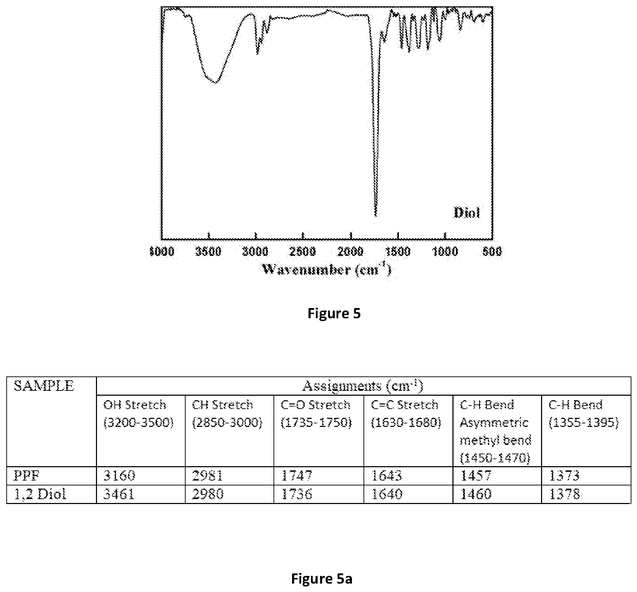

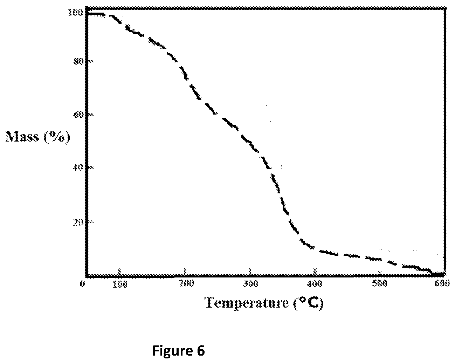

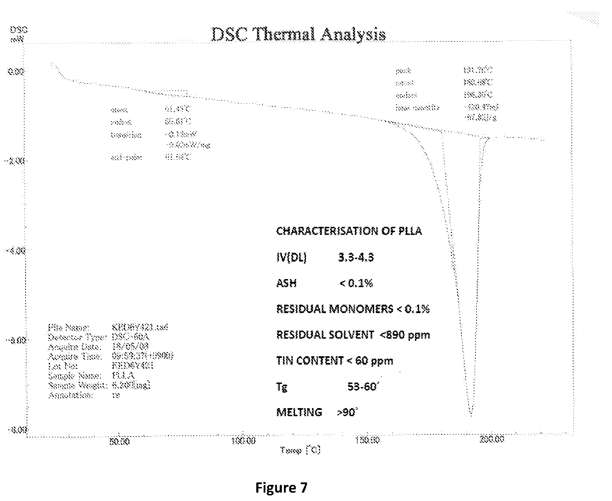

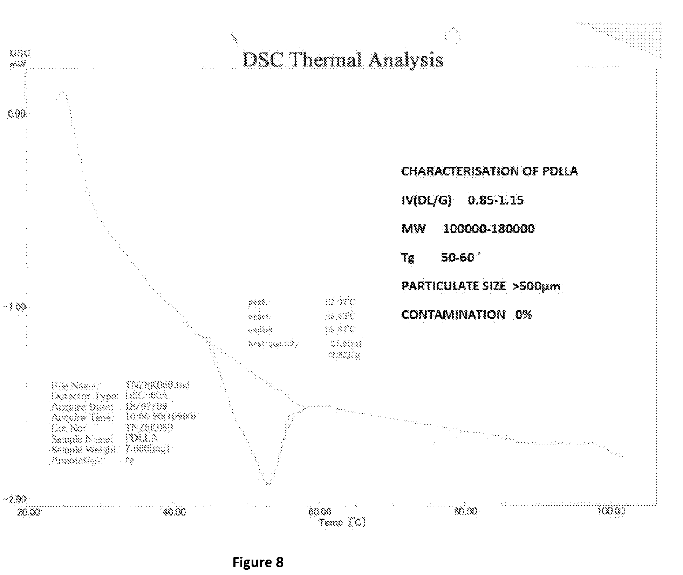

The bioinert and bioactive polymers were characterised for their properties. PPF and 1,2 Diol synthesized at the Department of Polymer Technology, Kamaraj College of Engineering and Technology, S.P.G.C. Nagar, K.Vellakulam-625 701, India. The medical grade PLA and PDLLA were procured from BioDegmer® Japan. The structural characterization (FTIR-8400S spectrophotometer, Shimadzu, Japan) and thermal evaluation (TA instruments DSC Q20) were carried out (Figure 3-8).

b) Assessment of the Effective Percentage of FP glass

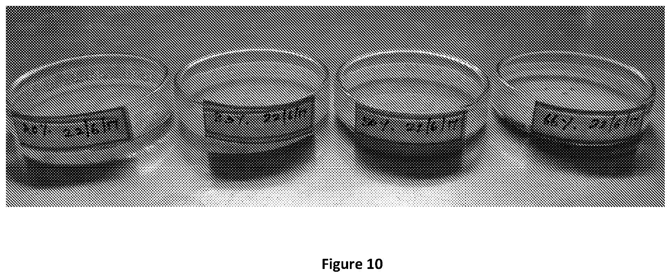

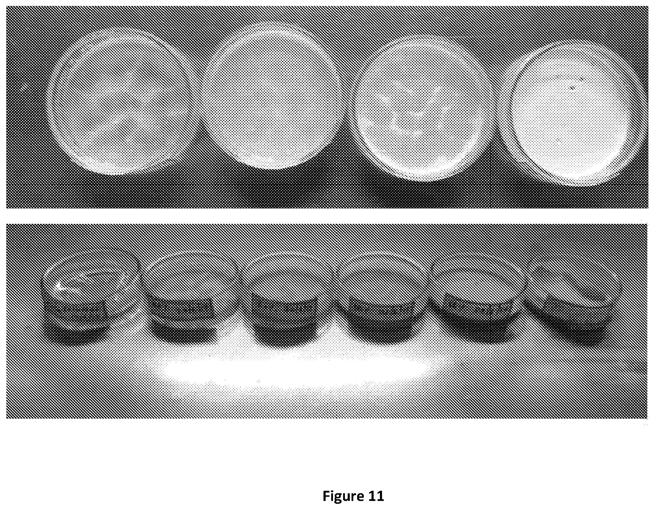

The optimum percentage of the FP glass was assessed by varying the proportions of the glass powder (0, 20, 33.3, 50, 66 and 75%) in the composite (Figure 10 & 11). The strength and ductility of the prepared material was examined manually. Also, the cell attachment was assessed in the composites as in the previous study to choose the right percentile of the glass powder (10, 12.5, 15, and 17.5 %) (Table 3 & 4)

Table 3

| S.NO. | PLA g(%) | PDLLA g(%) | PPF g(%) | GLASS POWDER g(%) |
|---|---|---|---|---|
| 1. | 5.6 (56) | 2.8 (28) | 0.6 (6) | 1 (10) |
| 2. | 6.15 (61.5) | 2.05 (20.5) | 0.525 (5.25) | 1.25 (12.5) |
| 3. | 6.46 (64.6) | 1.615 (16.15) | 0.425 (4.25) | 1.5 (15) |
| 4. | 6.736 (67.36) | 1.189 (11.89) | 0.325 (3.25) | 1.75 (17.5) |

Table 4

| Days | Samples (No. of cells attached on plate) | | | | Samples (No. of cells attached on Scaffold) | | | | Dead cells | | | |
|---|---|---|---|---|---|---|---|---|---|---|---|---|
| | 1 | 2 | 3 | 4 | 1 | 2 | 3 | 4 | 1 | 2 | 3 | 4 |
| 1 | 50 | 300 | 2000 | 48500 | 100 | 300 | 500 | 1000 | 49950 | 49700 | 48000 | 500 |
| 7 | 200 | 550 | 4500 | 69000 | 170 | 400 | 700 | 1500 | 49800 | 49450 | 45500 | 800 |
| 14 | 500 | 700 | 6000 | 80000 | 150 | 350 | 550 | 1200 | 49500 | 49300 | 44000 | 300 |
| 21 | 900 | 1000 | 11000 | 100000 | 100 | 300 | 500 | 1000 | 49100 | 49000 | 39000 | 350 |

c) Effect of Increasing the composition of the biopolymers

Of the four biopolymers PLA and PDLLA are bio inert and PPF and 1,2 Diol and FP GLASS are bioactive. The contribution of the bioactive ingredients were increased in minimal propositions at the cost of the bio inert PLA. The PLA share was reduced from 63.69% to 53.89% in graded decrements. It was substituted by increasing the FP GLASS and five types of scaffold were made. They were incubated with MG63 cell lines for 21 days following the previously mentioned protocol and the amount of adherent cells and the dead cells were tabulated. (Table 5 & 6)

Table 5

| S.NO. | PLA g(%) | PDLLA g(%) | PPF g(%) | GLASS POWDER g(%) |
|---|---|---|---|---|
| 1. | 7.005 (63.69) | 1.189 (10.81) | 0.33(3.00) | 2.475 (22.5) |
| 2. | 6.736 (61.24) | 1.189 (10.81) | 0.325(2.95) | 2.75(25) |
| 3. | 6.466 (58.78) | 1.189 (10.81) | 0.319(2.90) | 3.025 (27.5) |
| 4. | 6.197 (56.34) | 1.189 (10.81) | 0.314(2.85) | 3.30 (30) |
| 5. | 5.928 (53.89) | 1.189 (10.81) | 0.308(2.8) | 3.575(32.5) |

Table 6

| Days | Samples (No. of cells attached on plate) | | | | | Samples (No. of cells attached on Scaffold) | | | | | Dead cells | | | | |
|---|---|---|---|---|---|---|---|---|---|---|---|---|---|---|---|
| | 1 | 2 | 3 | 4 | 5 | 1 | 2 | 3 | 4 | 5 | 1 | 2 | 3 | 4 | 5 |
| 1 | 220 00 | 450 00 | 390 00 | 180 00 | 230 00 | 17 50 | 300 0 | 20 00 | 15 00 | 22 50 | 10 00 | 40 0 | 30 0 | 700 | 350 |
| 7 | 260 00 | 770 00 | 430 00 | 350 00 | 380 00 | 22 00 | 450 0 | 35 00 | 22 50 | 32 00 | 15 00 | 70 0 | 50 0 | 400 | 400 |
| 14 | 320 00 | 844 00 | 520 00 | 470 00 | 530 00 | 30 00 | 700 0 | 50 00 | 27 50 | 45 00 | 12 00 | 25 0 | 30 0 | 300 | 200 |
| 21 | 500 00 | 920 00 | 750 00 | 650 00 | 710 00 | 35 00 | 100 00 | 65 00 | 36 00 | 67 00 | 10 00 | 20 0 | 25 0 | 500 | 350 |

It was observed that the composites having 75% of glass were ductile and broke on bending. The composites with lesser percentage of the glass were not breaking and were elastic (Figure 10,11). When the polymer group had a small percentage (3%) of PPF/1,2 Diol the composite was not breaking and this expressed not only the bioactive nature of PPF/1,2 Diol but also the ability of it to alter the physical nature of the composite.

d) Effect of varying the percentage of the PLA and PDLLA with fixed PPF/1,2 Diol and FP glasses:

Scaffolds were made with keeping the proportion of the bioactive components PPF/1,2 Diol and FP glass as constant and the proportion of the bio inert components were varied, PLA being increased at the cost of reducing the percentage of PDLLA and the cell adhesion study and the no of viable and dead cells assessed as in the previous study and tabulated (Tables 7, 7a & 8, 8a)

Table 7

| S.NO. | PLA g(%) | PDLLA g(%) | PPF g(%) | GLASS POWDER g(%) |
|---|---|---|---|---|
| 1. | 6.406 (58.24) | 1.519 (13.81) | 0.325(2.95) | 2.75(25) |
| 2. | 6.516 (59.24) | 1.409 (12.81) | 0.325(2.95) | 2.75(25) |
| 3. | 6.626 (60.24) | 1.299(11.81) | 0.325(2.95) | 2.75(25) |
| 4. | 6.736 (61.24) | 1.189 (10.81) | 0.325(2.95) | 2.75(25) |
| 5. | 6.846 (62.24) | 1.079(9.81) | 0.325(2.95) | 2.75(25) |

Table 7a

| Days | Samples (No. of cells attached on plate) | | | | | Samples (No. of cells attached on Scaffold) | | | | | Dead cells | | | | |
|---|---|---|---|---|---|---|---|---|---|---|---|---|---|---|---|
| | 1 | 2 | 3 | 4 | 5 | 1 | 2 | 3 | 4 | 5 | 1 | 2 | 3 | 4 | 5 |
| 1 | 380 00 | 395 00 | 420 00 | 440 00 | 350 00 | 15 00 | 22 50 | 30 00 | 350 0 | 12 50 | 15 00 | 12 00 | 10 00 | 40 0 | 200 0 |
| 7 | 685 00 | 700 00 | 740 00 | 775 00 | 630 00 | 17 50 | 25 00 | 33 00 | 400 0 | 17 00 | 22 50 | 20 00 | 15 00 | 70 0 | 150 0 |
| 14 | 750 00 | 770 00 | 810 00 | 840 00 | 690 00 | 35 00 | 47 00 | 65 00 | 725 0 | 39 00 | 70 0 | 50 0 | 40 0 | 25 0 | 100 0 |
| 21 | 820 00 | 850 00 | 870 00 | 900 00 | 830 00 | 89 00 | 90 00 | 92 00 | 105 00 | 80 00 | 80 0 | 55 0 | 50 0 | 20 0 | 120 00 |

Table 8

| S.NO. | PLA g(%) | PDLLA g(%) | 1,2-Diol g(%) | GLASS POWDER g(%) |
|---|---|---|---|---|
| 1. | 6.406 (58.24) | 1.519 (13.81) | 0.325(2.95) | 2.75(25) |
| 2. | 6.516 (59.24) | 1.409 (12.81) | 0.325(2.95) | 2.75(25) |
| 3. | 6.626 (60.24) | 1.299(11.81) | 0.325(2.95) | 2.75(25) |
| 4. | 6.736 (61.24) | 1.189 (10.81) | 0.325(2.95) | 2.75(25) |
| 5. | 6.846 (62.24) | 1.079(9.81) | 0.325(2.95) | 2.75(25) |

Table 8a

| Days | Samples (No. of cells attached on plate) | | | | | Samples (No. of cells attached on Scaffold) | | | | | Dead cells | | | | |
|---|---|---|---|---|---|---|---|---|---|---|---|---|---|---|---|
| | 1 | 2 | 3 | 4 | 5 | 1 | 2 | 3 | 4 | 5 | 1 | 2 | 3 | 4 | 5 |
| 1 | 380 00 | 395 00 | 420 00 | 440 00 | 350 00 | 15 00 | 22 50 | 30 00 | 350 0 | 12 50 | 15 00 | 12 00 | 10 00 | 40 0 | 200 0 |
| 7 | 685 00 | 700 00 | 740 00 | 775 00 | 630 00 | 17 50 | 25 00 | 33 00 | 400 0 | 17 00 | 22 50 | 20 00 | 15 00 | 70 0 | 150 0 |
| 14 | 750 00 | 770 00 | 810 00 | 840 00 | 690 00 | 35 00 | 47 00 | 65 00 | 725 0 | 39 00 | 70 0 | 50 0 | 40 0 | 25 0 | 100 0 |
| 21 | 820 00 | 850 00 | 870 00 | 900 00 | 830 00 | 89 00 | 90 00 | 92 00 | 105 00 | 80 00 | 80 0 | 55 0 | 50 0 | 20 0 | 120 00 |

The cell adhesion to the scaffold and to the glass plate beneath by 1) Preparation of scaffold by varying the proportion of FP glass 2) Preparation of scaffold by increasing the percentage of bioactive components PPF/1,2 diol and FP GLASS at the cost of reducing the bioinert components 3) Preparation of scaffold by varying the percentage of the Bioinert components PLA and PDLLA keeping the Bioactive components fixed are provided in Tables 3,5,7,8.

The best results were achived when the bio active components (PPF/1,2 Diol +FP glass) were in the range of 10-30 % and the relatively bio inert components (PLA + PDLLA) were in the range of 54-67% (Table 4,6,7a and 8a.)

e) Biological Effect of Varying the Components:

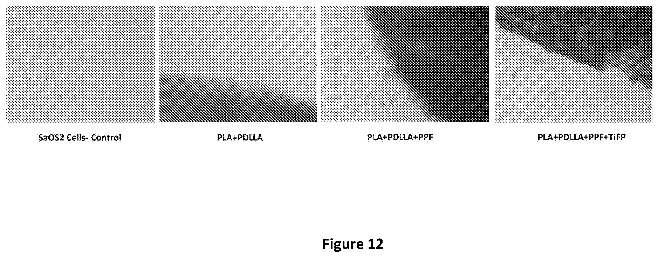

In order to assess how osteoblast-like cell line MG-63 adhere to scaffolds consisting of various combinations of polymers, Scaffolds of 1) PLA 2) PLA with PDLLA,3) PLA, PDLLA with PPF,4) PLA, PDLLA, PPF, with FP glass were made, cut into round shape of 12 mm using a cork borer, sterilized in UV, washed with PBS and immersed in serum-free MEM for an hour. Then scaffolds were transferred to 24 well plate and MG-63 cells ($5 X 10^4$) were seeded onto the scaffolds. Plates were incubated in $CO_2$ incubator with 5% $CO_2$ at 37°C for 1, 7, 14 and 21 days. Minimum essential media (MEM) supplemented with 10% foetal bovine serum (FBS), 50 U/mL penicillin, 50 mg/mL streptomycin 1% L-glutamine (Gibco) was used throughout the experiment. To avoid the nutrient exhaustion, for the wells with 7, 14 and 21 days incubation, media was changed every two days. After incubation, the wells with scaffolds were observed and recorded as photographs using phase contrast microscope (Olympus, Japan) with the camera attached software (Magnus Analytics Mag Vision software; version - x 64, 3.7.6820) for maximum number of attached cells. (Figure 12).

The evaluation conducted in MG63 cell lines underscores the bio activity of PPF and the FP glass. The cell adhesion which was nearly nil or negligible became obviously possible by the addition of a small quantity of PPF and was phenomenally increased by the addition of the FP glass. This effect is exhibited is shown in Figure 12.

**EXAMPLE 3: Porosity Assessment**

a) Membrane Integrity (Calcein AM) testing in relation to Pored/Unpored, +/-PPF/XPPF, +/-FP **glass**

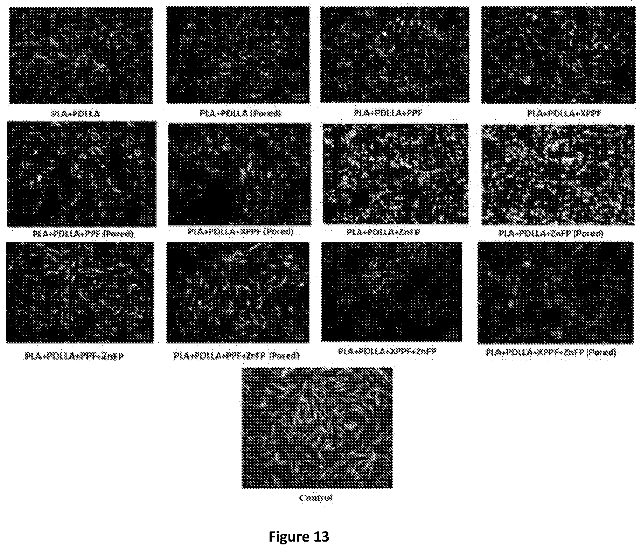

Twelve different scaffolds were fabricated to assess the significance of the porosity. The membrane integrity and cell morphology of the cells was evaluated by double staining. $1*10^5$ SaOS2 cells were incubated with the testing material (12 mm diameter scaffold) for 24h at 37° C with 5% $CO_2$. The cell supernatant medium was aspirated out. The scaffolds gently washed with ice cold PBS solution and finally 2μm Calcein AM added and incubated for 10m at 37° C. The cells were examined under a microscope. (Figure 13).

The calcein AM study to assess the cell wall integrity and the double staining to assess the cytotoxity showed interesting features. The control group of cells were not only brilliantly green but also showed homogenous spindle shape, indicating the integrity of cell wall and the metabolic potential. The addition of PPF to the basic components PLA+PDLLA increased the cell wall integrity and the addition of pores to the same increased the number of spindle shaped cells.

The addition of FP glass to the basic components PLA+PDLLA either pored are unpored increased the number of cells phenomenally but the quality of them were poor exhibited by their round shape rather than the spindle shape of the healthy cell.

When all the components PLA+PDLLA+PPF and FPglass were added both the intensity of fluorescence and the quality of the cells also increased and it was more so when pores were added to the composite.

The XPPF (auto polymerised PPF) when replaced the PPF in the composite there was only deleterious effect both in the fluorescence and the quality of the cells (Figure 13)

b) MTT Assayin relation to Pored/Unpored, +/-PPF/XPPF,+/- FP glass

The MTT assay was used to evaluate mitochondrial activity of live cells. Cells were seeded in 12-well plates containing test materials at density of $1 x 10^5$ cells/well in 100 μL complete medium/well and incubated for 24 h at 37 °C. After incubation, the cell culture media was aspirated, 10 μL MTT (5 mg/mL) was added to each well and incubated for 4 h. After wards, the resulting formazan crystals were solubilized in 100 μL/well of DMSO and quantified by measuring absorbance at 550 nm by Perkin Elmer microplate reader. Data were expressed as a percentage of control (untreated cells). (Table 9).

Table 9

| Sample name | Mean | SD | % cytotoxicity |
|---|---|---|---|
| PLA+PDLLA | 50.60 | 0.19 | 49.40 |
| PLA+PDLLA(Pored) | 48.21 | 1.21 | 51.79 |

(continued)

| Sample name | Mean | SD | % cytotoxicity |
|---|---|---|---|
| PLA+PDLLA+PPF | 43.70 | 0.78 | 56.30 |
| PLA+PDLLA+XPPF | 59.18 | 1.79 | 40.82 |
| PLA+PDLLA+PPF+ZnFP | 57.11 | 1.20 | 42.89 |
| PLA+PDLLA+XPPF+ZnFP | 42.70 | 1.36 | 57.30 |
| PLA+PDLLA+PPF(Pored) | 51.79 | 1.21 | 48.21 |
| PLA+PDLLA+XPPF(Pored) | 60.69 | 1.51 | 39.31 |
| PLA+PDLLA+PPF+ZnFP(Pored) | 78.37 | 3.92 | 21.63 |
| PLA+PDLLA+XPPF+ZnFP(Pored) | 69.53 | 1.71 | 30.47 |
| PLA+PDLLA+ZnFP | 54.23 | 0.47 | 45.77 |
| PLA+PDLLA+ZnFP(Pored) | 37.99 | 1.64 | 62.01 |

From the above study it can be inferred that the least toxic composite was that of PLA+ PDLLA+PPF+ FP glass (pored) (Table 9).

**EXAMPLE 4: Composite preparation and Scaffold Fabrication**

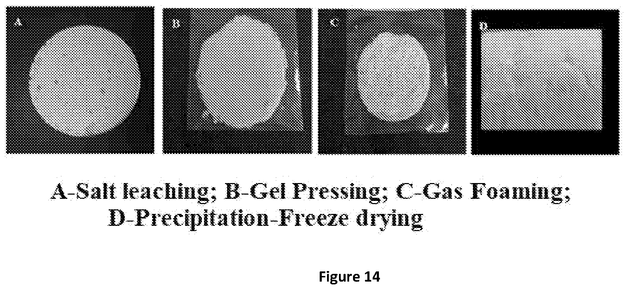

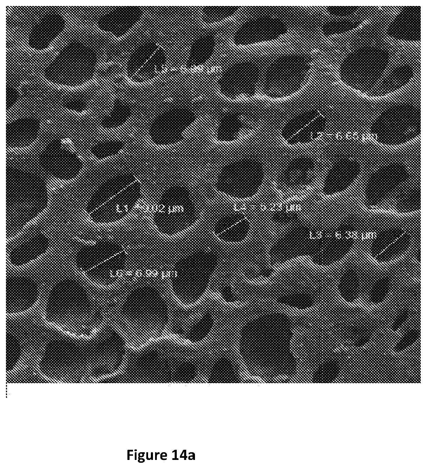

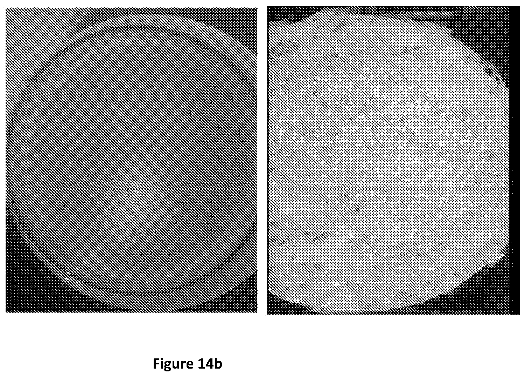

The four different methods were followed to prepare porous scaffold. (Salt leaching, Gas foaming, Gel pressing and Precipitation-Freeze Drying) (Figure 14).

SALT LEACHING:

The calculated amount of the PLA, PDLLA, PPF/Diol and AgFP/ZnFP/MgFP were taken and mixed with dichloromethane. The porogen (Sucrose-$C_{12}H_{22}O_{11}$) was sieved in the 300 and 100 $\mu$ mesh and it was added in 30% V/V basis. The porogen was mixed with the mixture using magnetic stirrer at 300rpm. This slurry was poured into a teflon film coated petri dish and was placed in a warm chamber for 24 h. After drying, the film was compressed at 70°C for 10 m. By sonication, the porogen was leached out using double distilled water. The prepared scaffold was dried in laminar air hood.

GAS FOAMING

The calculated amount of the PLA, PDLLA, PPF/Diol and AgFP/ZnFP/MgFP were taken and mixed with dichloromethane. The porogen (Ammonium bicarbonate- $NH_4)HCO_3$) was sieved in the 300 and 100 $\mu$ mesh and it was added in 30% V/V basis. The porogen was mixed with the mixture using magnetic stirrer at 300rpm. This slurry was poured into a teflon film coated petri dish and was placed in a warm chamber for 24 h. After drying, the film was immersed in hot water, $CO_2$ emission occurred which inturn generates pores. Once all the bubbles settle down, the scaffold was placed in ice cold ethanol for 2m. The fabricated scaffold was dried under laminar air hood for 24h.

GEL PRESSING

The calculated amount of the PLA, PDLLA, PPF/Diol and AgFP/ZnFP/MgFP were taken and mixed with dichloromethane. The porogen (Sucrose-$C_{12}H_{22}O_{11}$) was sieved in the 300 and 100 $\mu$ mesh and it was added in 30% V/V basis. The porogen was mixed with the mixture using magnetic stirrer at 300rpm. This slurry was poured into a teflon film coated petri dish and was placed in a warm chamber for 24 h. After complete evaporation of the solvent, the two films were pasted with methylene chloride and it was compressed at 70°C for 10 minutes. By sonication, the porogen was leached out using double distilled water. The prepared scaffold was dried in laminar air hood.

PRECIPITATION-FREEZE DRYING

The calculated amount of the PLA, PDLLA, PPF/Diol and AgFP/ZnFP/MgFP was taken and mixed with dichloromethane. The solution was slowly poured in to ice cold ethanol (nonsolvent) under efficient stirring. The fibril like precipitate was obtained and it was washed with the double distilled water. The precipitate was packed into the

cylindrical tube. The obtained precipitate was centrifuged at 3000rpm for 15m and it was kept in freezer for 12h.The scaffold was freeze dried for 8h.

GEL FOAM CASTING UNDER RAPID HEATING

The required amount of the PLA, PDLLA, PPF/Diol and AgFP/ZnFP/MgFP were taken and mixed with dichloromethane under magnetic stirrer at 300rpm. Once the mixture homogenised, the composite was slowly poured over a hot glass plate (70°C). The solution started to boil and emitted the dichloromethane. With solvent evaporation, random pores were generated. The constant, continuous boiling kept the composite homogenous in spite of the difference in the densities of the four components. After complete evaporation, highly interconnected porous scaffold with homogenous distribution of the components was obtained. (Figure 14 a, 14b). The scaffold thus made can be marsealised to a powder, or cut into strips, or rolled into a cylinder. The same procedure were repeated with different solvents acetone, toluene, and chloroform and the same result was achieved.

The essential problem in homogenising the components was that all the three polymers chosen were soluble only in organc solvents and the essential bioactive inorganic component was highly hydrophilic and was soluble only in water. The other problem faced in homogenising the components was the gross difference in their densities. The other pre requiste apart from homogenesity was the essential need of pores and interconnecting pores for better bioactivity. The convenentional methods like salt leaching,gas leaching, gel pressing, precipitation and freeze drying all failed to achive the desired homogenesity and the porosity. The highly dense FPglass powder setteled in the base layer of the composite (Figure 14). Also at the end of all these procedures the pores which had been designed to be aroung 200 microns got squeezed to around 10 microns because of the compression (Figure 14a). But when Gel foam casting and rapid heating ,two methods were combined the constant boiling gave the needed homogenesity and the vaporisation of the solvent gave the desired pores (Figure 14b).

The following composites were prepared according to the Gel Foam Casting under Rapid Heating mentioned above and evaluated.

Table 10 a

| Composite no | PLA | PDLLA | PPF | 1,2 DIOL | ZnFP | MgFP | AgFP |
|---|---|---|---|---|---|---|---|
| 1 | √ | x | x | √ | x | x | √ |
| 2 | √ | √ | x | √ | x | x | √ |
| 3 | √ | x | √ | x | x | x | √ |
| 4 | √ | √ | √ | x | x | x | √ |
| 5 | √ | x | x | √ | √ | x | x |
| 6 | √ | √ | x | √ | √ | x | x |
| 7 | √ | x | √ | x | √ | x | x |
| 8 | √ | √ | √ | x | √ | x | x |
| 9 | √ | x | x | √ | x | √ | x |
| 10 | √ | √ | x | √ | x | √ | x |
| 11 | √ | x | √ | x | x | √ | x |
| 12 | √ | √ | √ | x | x | √ | x |

**Table 10b**

| Scaffolds | PLA (%) | PDLLA (%) | DIOL (%) | PPF (%) | AgFp/ ZnFp/MgFp (%) |
|---|---|---|---|---|---|
| PLA+Diol+AgFp | 64 | - | 8.5 | - | 27.5 |
| PLA+PDLLA+Diol+AgFp | 59.69 | 9.81 | 5.0 | - | 25.5 |
| PLA+PPF+AgFp | 64 | - | - | 8.5 | 27.5 |
| PLA+PDLLA+PPF+ AgFp | 59.69 | 9.81 | - | 5.0 | 25.5 |
| PLA+Diol+ZnFp | 64 | - | 8.5 | - | 27.5 |
| PLA+PDLLA+Diol+ZnFp | 59.69 | 9.81 | 5.0 | - | 25.5 |

(continued)

| Scaffolds | PLA (%) | PDLLA (%) | DIOL (%) | PPF (%) | AgFp/ ZnFp/MgFp (%) |
|---|---|---|---|---|---|
| PLA+PPF+ZnFp | 64 | - | - | 8.5 | 27.5 |
| PLA+PDLLA+PPF+ ZnFp | 59.69 | 9.81 | - | 5.0 | 25.5 |
| PLA+Diol+MgFp | 64 | - | 8.5 | - | 27.5 |
| PLA+PDLLA+Diol+MgFp | 59.69 | 9.81 | 5.0 | - | 25.5 |
| PLA+PPF+MgFp | 64 | - | - | 8.5 | 27.5 |
| PLA+PDLLA+PPF+MgFp | 59.69 | 9.81 | - | 5.0 | 25.5 |

**EXAMPLE 5: Cytotoxic evaluation of the fabricated composites as scaffolds:**

MTT Assay:

The non-toxic nature of the fabricated scaffolds were assessed by Saos-2 cell line (ATCC-85). $5x10^6$ SaOS2 cells at passage 25 were incubated in control medium supplemented with 10% fetal bovine serum 200 mM L-glutamine, 10 mM ascorbic acid, β-phosphate, 100 U/mL penicillin and 100 μg/mL streptomycin. The cells were incubated for 24-48h for confluency. The confluent SaOS2 cells were washed twice using 1X PBS. A dimension of 2X2 $cm^2$ of scaffold were placed in six well plates and incubated for 48h.The morphology of the cells was observed under an inverted microscope. The scaffolds were removed carefully and MTT was added and incubated for 4h. The resulting formazan crystal was dissolved using DMS. The OD values were measured at 405nm in a micro plate reader and the reading was tabulated. (Table 11).

Table 11

| Composites | % of live cells |
|---|---|
| PLA+Diol+AgFP | 90.95 |
| PLA+PDLLA+Diol+AgFP | 79.84 |
| PLA+PPF+AgFP | 81.48 |
| PLA+PDLLA+PPF+AgFP | 81.13 |
| PLA+Diol+ZnFP | 89.72 |
| PLA+PDLLA+Diol+ZnFP | 84.36 |
| PLA+PPF+ZnFP | 93.42 |
| PLA+PDLLA+PPF+ZnFP | 57.20 |
| PLA+Diol+MgFP | 90.54 |
| PLA+PDLLA+Diol+MgFP | 81.48 |
| PLA+PPF+MgFP | 53.09 |
| PLA+PDLLA+PPF+MgFP | 86.42 |
| Control | 100 |

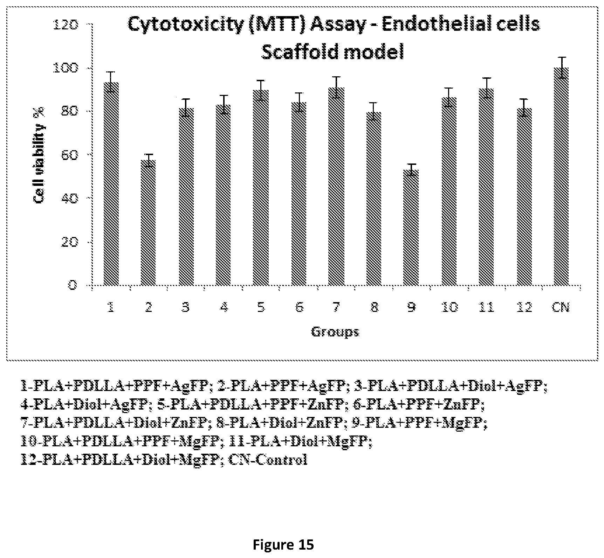

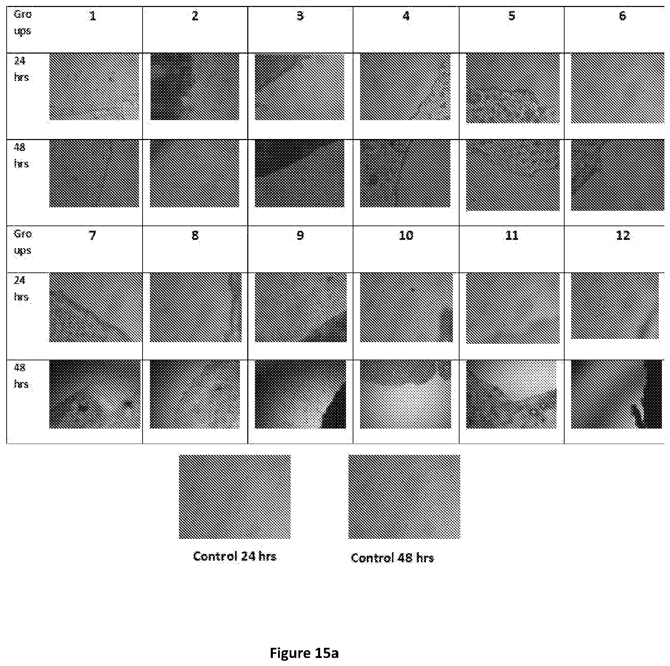

The same procedure was done with human endothelial cell lines and the results were noted with the morphological changes of the cells (Figure 15 and 15a).

The cytotoxicty of composites assessed by cytotoxic evaluation in both human endothelial cell lines and SaOS2 cell lines. With the HE cell lines except two composites (PLA+PPF+AgFP and PLA+PPF+ZnFP) all other composites showed viability more than 80% (Figure 15, 15a). With SaOS2 cell lines except two composites (PLA+PDLLA+PPF+ZnFP and PLA+PPF+MgFP ) all the other 10 had viabilty above 80%. (Table 11).

**EXAMPLE 6: Biological evaluation of the fabricated composites as scaffolds**

1)Alkaline Phosphatase (ALP) activity of the scaffolds

A 1* $10^6$ MG63 cells were plated in culture plates and incubated for 48 h at 37 °C in 5 % $CO_2$ incubator. Once the cells were confluent, it was treated with 2cm* 2cm of each scaffold sample one in each well and incubated. After incubating for 48 h, cells were washed twice with ice cold PBS and homogenized in 50μL assay buffer. The insoluble materials were centrifuged at 13,000 rpm for 3 min. The test samples with different concentrations of the exudates were added into 96-well plate and then 10μL of ALP was added to each well. Then,50μL of the 5mM pNPP solution was added to each well containing the test samples. The reaction mixture was incubated for 60 minute at 25 °C in dark condition. A 20 μL stop solution was added to terminate the ALP activity in the sample. The OD values are measured at 405nm in a micro plate reader and the obtained results are noted in table. (Table 12).

Table 12

| Composites | ALP (IU/mL) |
|---|---|
| PLA+Diol+AgFP | 0.443 |
| PLA+PDLLA+Diol+AgFP | 1.915 |
| PLA+PPF+AgFP | 0.596 |
| PLA+PDLLA+PPF+AgFP | 1.406 |
| PLA+Diol+ZnFP | 0.938 |
| PLA+PDLLA+Diol+ZnFP | 2.306 |
| PLA+PPF+ZnFP | 0.503 |
| PLA+PDLLA+PPF+ZnFP | 1.543 |
| PLA+Diol+MgFP | 1.081 |
| PLA+PDLLA+Diol+MgFP | 1.208 |
| PLA+PPF+MgFP | 1.39 |
| PLA+PDLLA+PPF+MgFP | 1.544 |
| Control | 1.529 |

The ability of the fabricated composites to enhance the secretion of Alkaline phosphotase (ALP) in the SaOS2 cell lines was evalualted. ALP is a vital factor involved in most of the stages of Bone tissue formation. The values obtained showed that only two composites with 1,2,Diol and AgFP/ZnFP showed more secretion than the control while the composites with PPF were just at par with the control. The composites with MgFP,all the four types of them showed lower activity than that of the control (Table 12).

2)Evaluation of Osteocalcin, Collagen II, Run X2 secretion of the fabricated composites as scaffolds by RT PCR

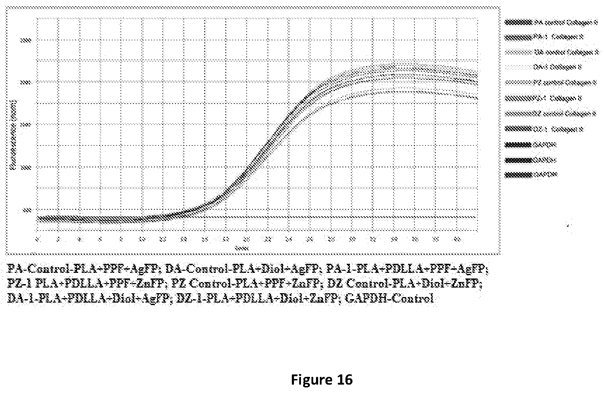

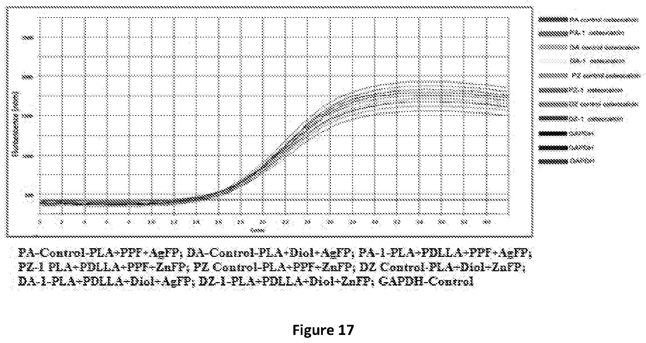

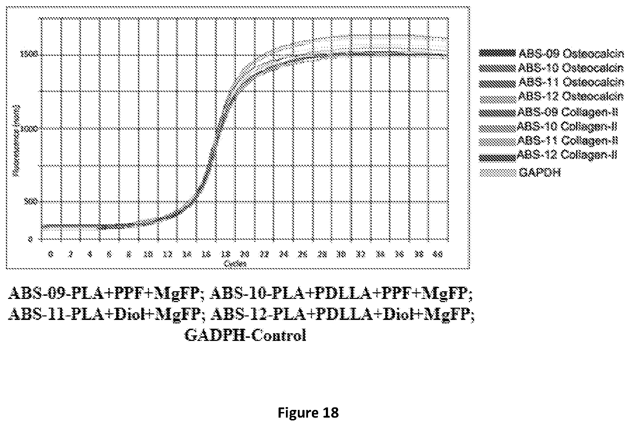

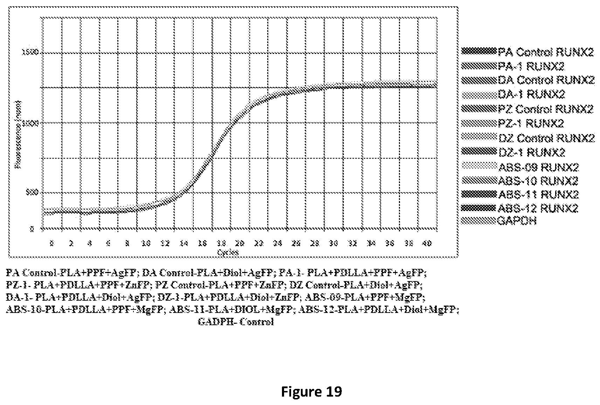

The study was done in SaOS2 cell lines. The total RNA was isolated from osteoblasts using TRIzol™ Reagent according to the manufacturer's protocol. The concentration of RNA was determined at 260/280 nm using NanoDrop spectrophotometer. For reverse transcription polymerase chain reaction (RT-PCR) the cDNA was synthesized by SuperScriptTM First-Strand Synthesis System (Thermo Scientific) following the instructions provided. The synthesized cDNA was stored at 20 °C for later use. Simultaneous gene expression level for COL II (Figure 16 & 18), OCN (Figure 17 & 18), and Runx2 (Figure 19) genes were measured by RT-PCR using SYBR green method.

Cycling program of temperature and time

| Phase | Duration (min) | Temperature(°C) | Number of cycles |
|---|---|---|---|
| Hold | 2 | 50 | 1 |
| Initial Denaturation | 10 | 95 | 1 |
| Denaturation, Annealing | 0.25,0.5,0.5 | 95,60,72 | 40 |

(continued)

| Phase | Duration (min) | Temperature(°C) | Number of cycles |
|---|---|---|---|
| Extension | 10 | 72 | 1 |

A real-time PCR reaction mixture (50 μL) preparation protocol followed as 25 μL SYBR Green Mix (2x), 0.5 μL liver cDNA, 2 μl primer pair mix (5 pmol/μL each primer) and 22.5 μL $H_2O$ The primers used for PCR were as follows:

Collagen type II:

Forward primer: CATGAGGGCGCGGTAGAGA
Reverse Primer: ATCCCCTCTGGGTCCTTGTT
Product length : 296

Osteocalcein

Forward primer : TCACACTCCTCGCCCTATTG
Reverse Primer : CTCTTCACTACCTCGCTGCC
Product length : 132

Runx2Sequence (5'->3') Template strand Length Start Stop Tm GC% Self complementarity Self 3' complementarity

Forward primer CCACCGAGACCAACAGAGTC Plus
Reverse primer GTCACTGTGCTGAAGAGGCT
Product length 119.

The analysis of the results was performed using ABI PRISM® 7000 Sequence DetectionSystem software that enables more sensitive and accurate estimation of the relative gene expression. The results were tabulated. (Table 13).

Table 13

| Composites | MTT | ALP (IU/mL ) | Collagen II (Fold increase) | Osteocalcin (Fold increase) | Run X2 (Fold increase) | Chondroitin levels (ng/mL) |
|---|---|---|---|---|---|---|
| PLA+Diol+AgFP | 90.95 | 0.443 | 0.16 | 2.3 | 0.04 | 7.45 |
| PLA+PDLLA+Diol+A gFP | 79.84 | 1.915 | 0.81 | 4.7 | 0.55 | 11.54 |
| PLA+PPF+AgFP | 81.48 | 0.596 | 0.4 | 0.22 | 0.03 | 3.25 |
| PLA+PDLLA+PPF+ AgFP | 81.13 | 1.406 | 4.4 | 3.2 | 0.5 | 5.43 |
| PLA+Diol+ZnFP | 89.72 | 0.938 | 0.12 | 0.07 | 0.01 | 10.21 |
| PLA+PDLLA+Diol+ ZnFP | 84.36 | 2.306 | 1.7 | 1.2 | 0.4 | 3.85 |
| PLA+PPF+ZnFP | 93.42 | 0.503 | 0.5 | 1.4 | 0.09 | 13.23 |
| PLA+PDLLA+PPF+ ZnFP | 57.20 | 1.543 | 1.1 | 2.6 | 0.98 | 9.78 |
| PLA+Diol+MgFP | 90.54 | 1.081 | 0.25 | 0.05 | 0.78 | 10.88 |
| PLA+PDLLA+Diol+ MgFP | 81.48 | 1.208 | 0.93 | 0.19 | 0.78 | 8.21 |
| PLA+PPF+MgFP | 53.09 | 1.39 | 0.23 | 0.23 | 0.21 | 5.43 |
| PLA+PDLLA+PPF+ MgFP | 86.42 | 1.544 | 0.53 | 0.24 | 0.2 | 10.21 |
| Control | 100 | 1.529 | | | | 0.18 |

The three essential gene markers in the synthesis of bone from the stage of mesenchymal stemcells to that of the osteocyte maturation are OSTEOCALCIN, COLLAGEN II, and RUN-X2. When the results were charted to scrutinise the fold change than the control,the fold increase in collagen II was highest with PLA+PDLLA+ PPF+AgFP and the highest fold increase in osteocalcin was also with AgFP but when constituted with 1,2,Diol than with PPF. The highest fold change in

RUN_X2 than the control was with ZnFP when combined with PLA+PDLLA+PPF . All the Mg based composites fared poorly with all the three types of gene markers. (Table 13, Figures 16-19)

c)Chondroitin sulphate assay of the scaffolds:

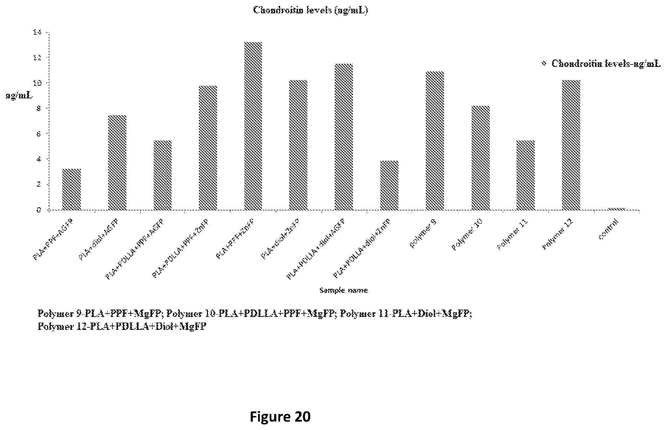

SaOS2 cell line was inoculated with the various composites for 48 hours. The cells were washed three times in cold PBS and suspended againin PBS (1x), frozen cells at ≤ -20°C and thawed. Repeated the freeze/thaw cycle 3 times.) Centrifuge at 1,500 x g for 10minutes at 2-8°C to remove cellular debris. Chondroitin sulphate was measured using competitive ELISA method (Robonik, India). (Figure 20) (Table 14).

Table 14

| Composites | Chondroitin levels (ng/mL) |
|---|---|
| PLA+Diol+AgFP | 7.45 |
| PLA+PDLLA+Diol+AgFP | 11.54 |
| PLA+PPF+AgFP | 3.25 |
| PLA+PDLLA+PPF+AgFP | 5.43 |
| PLA+Diol+ZnFP | 10.21 |
| PLA+PDLLA+Diol+ZnFP | 3.85 |
| PLA+PPF+ZnFP | 13.23 |
| PLA+PDLLA+PPF+ZnFP | 9.78 |
| PLA+Diol+MgFP | 10.88 |
| PLA+PDLLA+Diol+MgFP | 8.21 |
| PLA+PPF+MgFP | 5.43 |
| PLA+PDLLA+PPF+MgFP | 10.21 |
| Control | 0.18 |

The results showed all the twelve composites showed many fold increase in the secretion of CS than the control, immaterial of the component having 1,2,Diol or PPF and the FPglass being either Ag, Zn, or Mg.(Table 14).

**EXAMPLE 7: *In Vitro* Evaluation of the Scaffolds:**

1)By following standard kokubo protocol, simulated body fluid (SBF) was prepared. All the fabricated scaffolds were cut into 2*2cm$^2$ size. The scaffolds were placed in 20 mL SBF filled glass container, for a period of 21 days at 5% $CO_2$ incubator (Heraus- Germany). The pH variation was noted everyday using pH meter E1 model. After 21 days, the scaffolds were carefully removed; dried in laminar air flow for 48h.The variation in the pH over 21 days of all the specimen were charted. (Table 15)

Table 15

| Samples | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| PLA_PPF_ZnFp | 6.63 | 6.64 | 7 | 7 | 6.91 | 6.97 | 6.93 | 7 | 7 | 7 | 7 | 7 | 6.85 | 6.84 | 6.77 | 7 | 6.87 | 7 | 6.83 | 6.75 | 6.77 |
| PLA_PDLLA_PPF_ZnFp | 6.5 | 6.7 | 7 | 7 | 7 | 6.93 | 6.93 | 7 | 7 | 7 | 7 | 7 | 6.88 | 6.72 | 6.78 | 7 | 7 | 6.92 | 6.75 | 6.74 | 6.72 |
| PLA_PPF_AgFp | 6.6 | 6.76 | 7 | 7 | 7 | 7 | 7 | 7 | 7 | 7 | 7 | 7 | 6.87 | 6.75 | 6.88 | 7 | 7 | 6.82 | 6.78 | 6.75 | 6.78 |
| PLA_PDLLA_PPF_AgFp | 6.7 | 6.82 | 7 | 7 | 7 | 7 | 7 | 7 | 7 | 7 | 7 | 7 | 6.9 | 6.75 | 6.8 | 7 | 7 | 6.84 | 6.75 | 6.76 | 6.8 |
| PLA_Diol_ZnFp | 6.57 | 6.79 | 7 | 7 | 7 | 7 | 7 | 7 | 7 | 7 | 7 | 7 | 6.82 | 6.71 | 6.82 | 7 | 7 | 6.82 | 6.75 | 6.75 | 6.78 |
| PLA_PDLLA_Diol_ZnFp | 6.7 | 6.74 | 7 | 7 | 7 | 6.92 | 6.93 | 7 | 7 | 7 | 7 | 7 | 6.87 | 6.71 | 6.81 | 7 | 7 | 6.82 | 6.75 | 6.72 | 6.73 |
| PLA_Diol_AgFp | 6.66 | 6.76 | 7 | 7 | 7 | 7 | 7 | 7 | 7 | 7 | 7 | 7 | 6.93 | 6.77 | 6.78 | 7 | 6.92 | 6.92 | 6.76 | 6.75 | 6.76 |
| PLA_PDLLA_Diol_AgFp | 6.71 | 6.78 | 7 | 7 | 7 | 7 | 7 | 7 | 7 | 7 | 7 | 7 | 6.89 | 6.73 | 6.8 | 7 | 7 | 6.86 | 6.77 | 6.78 | 6.78 |
| PLA_PPF_MgFp | 6.54 | 6.67 | 7 | 7 | 6.89 | 6.93 | 6.88 | 7 | 7 | 6.9 | 7 | 6.92 | 6.79 | 6.64 | 6.68 | 6.89 | 6.81 | 6.74 | 6.69 | 6.68 | 6.66 |
| PLA_PDLLA_PPF_MgFp | 6.53 | 6.76 | 7 | 7 | 6.85 | 6.9 | 6.89 | 6.93 | 6.91 | 6.92 | 6.92 | 6.92 | 6.8 | 6.64 | 6.69 | 6.89 | 6.83 | 6.74 | 6.68 | 6.69 | 6.7 |
| PLA_Diol_MgFp | 6.65 | 6.75 | 7 | 7 | 6.97 | 6.97 | 6.97 | 7 | 7 | 7 | 7 | 7 | 6.83 | 6.71 | 6.75 | 7 | 7 | 6.85 | 6.74 | 6.77 | 6.76 |
| PLA_PDLLA_Diol_MgFp | 6.6 | 6.72 | 7 | 6.93 | 6.84 | 6.82 | 6.81 | 6.89 | 6.88 | 6.87 | 6.89 | 6.88 | 6.71 | 6.62 | 6.69 | 6.89 | 6.83 | 6.72 | 6.66 | 6.67 | 6.65 |
| PLA_PDLLA_PPF_ZnFp(RH) | 6.74 | 6.82 | 7 | 7 | 6.93 | 7 | 7 | 7 | 7 | 7 | 7 | 7 | 6.79 | 6.65 | 6.74 | 7 | 6.93 | 6.81 | 6.71 | 6.73 | 6.71 |
| PLA_PDLLA_PPF_AgFp(RH) | 6.77 | 6.84 | 7 | 7 | 6.93 | 6.93 | 6.92 | 7 | 7 | 7 | 7 | 7 | 6.79 | 6.68 | 6.74 | 7 | 6.93 | 6.83 | 6.72 | 6.74 | 6.69 |
| PLA_PDLLA_PPF_MgFp(RH) | 6.76 | 6.85 | 7 | 7 | 6.91 | 6.91 | 6.9 | 7 | 6.92 | 6.93 | 7 | 6.93 | 6.78 | 6.68 | 6.61 | 6.89 | 6.84 | 6.76 | 6.66 | 6.66 | 6.65 |
| Single Layer | 6.9 | 7 | 7 | 7 | 6.92 | 7 | 7 | 7 | 7.1 | 7.09 | 7.09 | 7 | 7.15 | 7 | 6.93 | 7 | 7 | 7.07 | 7 | 7 | 7 |
| Multi Layer | 6.79 | 6.89 | 6.9 | 7 | 7 | 6.84 | 6.88 | 6.84 | 7 | 7 | 7 | 7 | 7 | 6.83 | 6.84 | 6.8 | 6.86 | 7 | 6.86 | 6.83 | 6.86 |

The pH variation of all the compression moulded specimens showed uniformly a reduction in the first 2 days which is because of phosphoric acid formation. And all the specimens bounced back to 7 on the third day due to the alkaline earth metal ($Na^+$ and/or $Ca^{2+}$) release. The dissolution of the ions thus replaces $H^+$ ions by cations ($Na^+$ and/or $Ca^{2+}$) leading to an increase in hydroxyl ion concentration. None of them went below 6.5 even in the first two days. From then on it showed a steady variation between 7 and 6.7.

The scaffolds, single layered strip and the multi layered cylinders made by Gel Foam Casting under rapid heating showed a better pHeven in the first two days and never went below 6.8 and the end stage also showed higher pHthan the compression moulded scaffolds. The highest pH reached was with the strip of scaffold made by rapid heating method and it was 7.15. This variation shows the better homogenesity and the porosity achived by the rapid heating method which avoids high acidic environment that can lead on to rejection (Table 15).

2) XRD

The pre and post immersion specimen (pre and post immersion refers to the scaffolds before immersion in the SBF and after immersion in SBF and drying) was subjected to XRD evaluation. The X-Ray Diffraction was captured using PANalyticalX'PertPRO powder X-ray Diffractometer The deposited materials crystal size was calculated semi-quantitatively by adopting Scherrer equation.

$$D=k\,\lambda/\beta\cos\theta$$

where:

D is the mean size of the ordered (crystalline) domains, which may be smaller or equal to the grain size, which may be smaller or equal to the particle size; (nm)
k is a dimensionless shape factor, with a value close to unity. The shape factor has a typical value of about 0.9, but varies with the actual shape of the crystallite;
$\lambda$ is the X-ray wavelength; $\lambda$=0.15406nm
$\beta$ is the line broadening at half the maximum intensity (FWHM), after subtracting the instrumental line broadening, in radians.
$\theta$ is the Bragg angle. (Table 16)

Table 16

| Samples | Pre immersion (crystal size nm) | Post immersion (crystal size nm) |
|---|---|---|
| PLA_PPF_ZnFP | 16.46 | 23.32 |
| PLA_PDLLA_PPF_ZnFP | 17.45 | 21.86 |
| PLA_PPF_AgFP | 14.07 | 22.60 |
| PLA_PDLLA_PPF_AgFP | 34.54 | 15.92 |
| PLA_Diol_ZnFP | 16.35 | 21.90 |
| PLA_PDLLA_Diol_ZnFP | 29.45 | 18.33 |
| PLA_Diol_AgFP | 16.11 | 28.62 |
| PLA_PDLLA_Diol_AgFP | 19.27 | 22.64 |
| PLA_PPF_MgFP | 21.44 | 20.74 |
| PLA_PDLLA_PPF_MgFP | 13.22 | 16.33 |
| PLA_Diol_MgFP | 25.86 | 20.71 |
| PLA_PDLLA_Diol_MgFP | 11.57 | 14.65 |

The XRD of the three specimens prepared by combining rapid heating and gel foam casting was evaluated for the pre immersion and the post immersion status. (Table 17).

Table 17

| Samples | Pre immersion (crystal size nm) | Post immersion (crystal size nm) |
|---|---|---|
| PLA_PDLLA_PPF_ZnFP(RH) | 22.49 | 20.49 |
| PLA_PDLLA_PPF_AgFP(RH) | 30.81 | 13.94 |
| PLA_PDLLA_PPF_MgFP(RH) | 19.87 | 14.24 |
| STRIP (RH) | 26.47 | 50.29 |
| CYLINDER (RH) | 33.01 | 39.66 |

In the XRD evaluation, the crystal size(D)of the deposited material was calculated in both the pre immersion and the post immersion status by Schrrer equation. Among the pre immersion values, the crystal size of the PLA+PDLLA+PPF+AgFP showed the highest value. This is arributed to the high hydrophilicity of the composite and the reaction it has undergone with the atmospheric humidity during the waiting period of scaffolding and the evaluation.(Table 16) The same effect is observed in the scaffolds made by Gel foam casting under rapid heating method also and the highest size of the crystals were seen with the same composite of AgFP (Table 17).

3) FITR analysis:

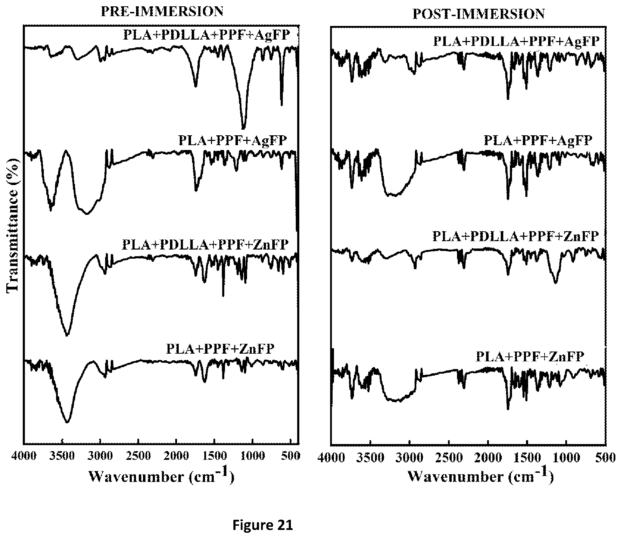

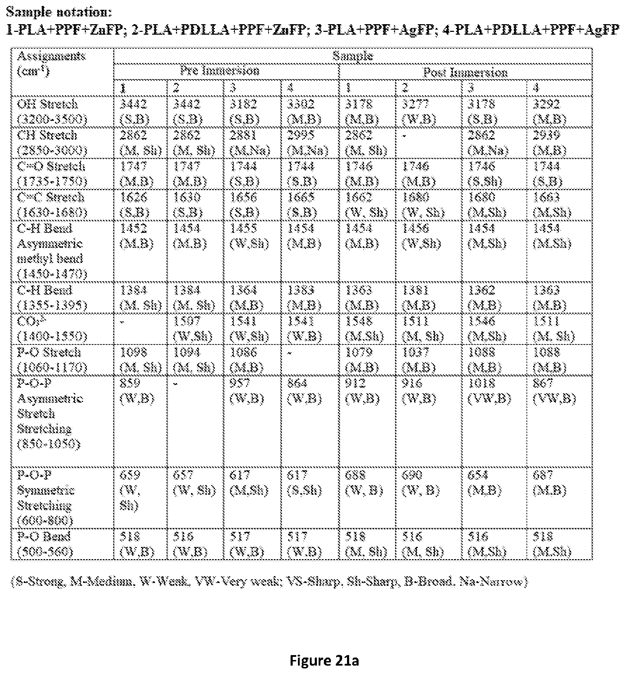

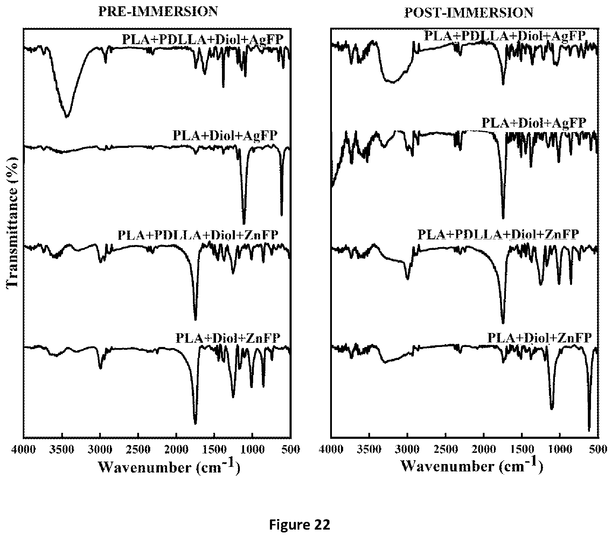

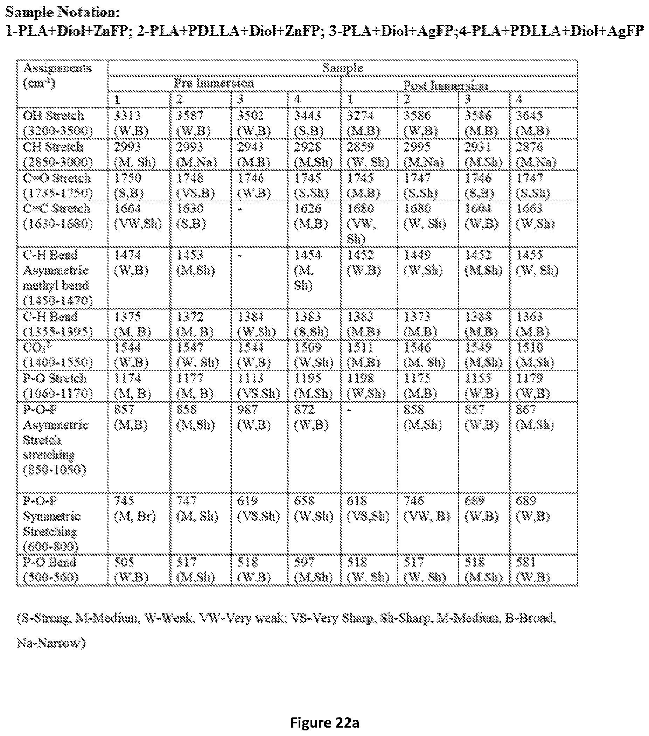

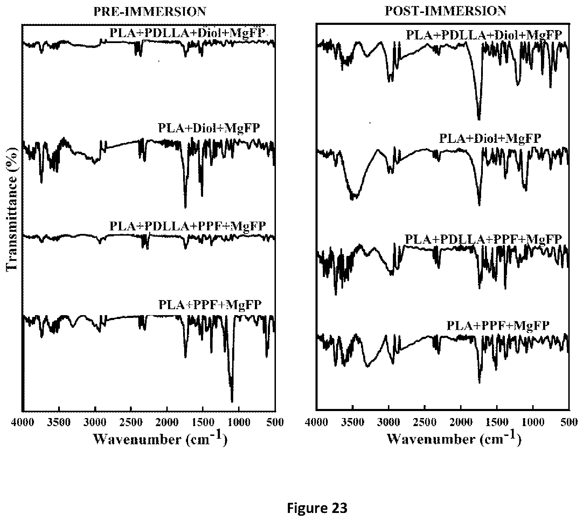

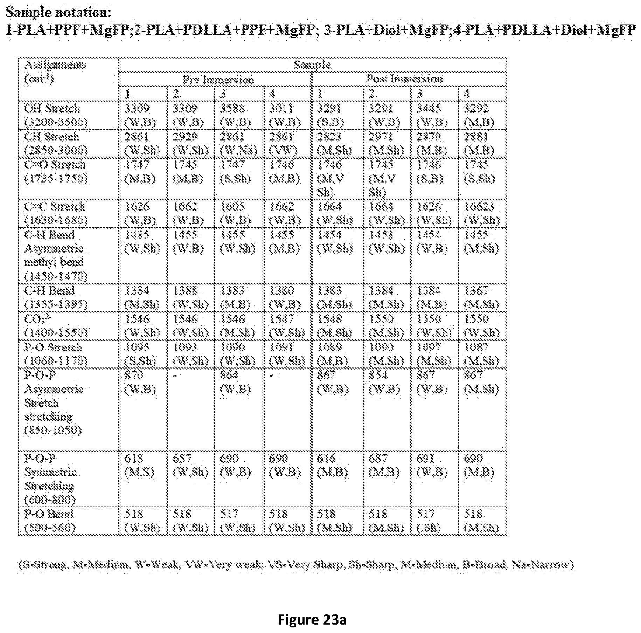

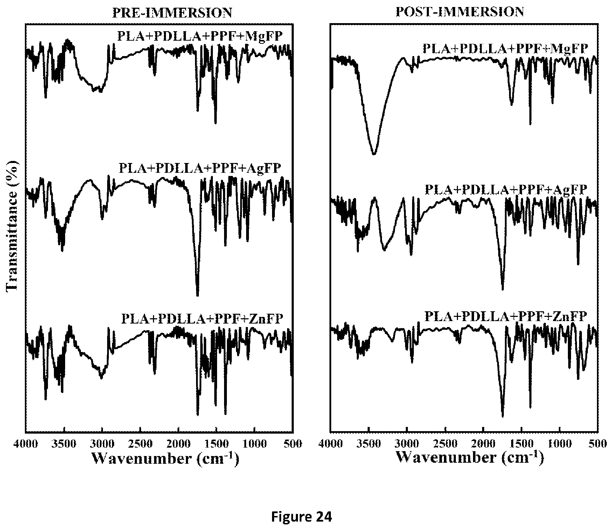

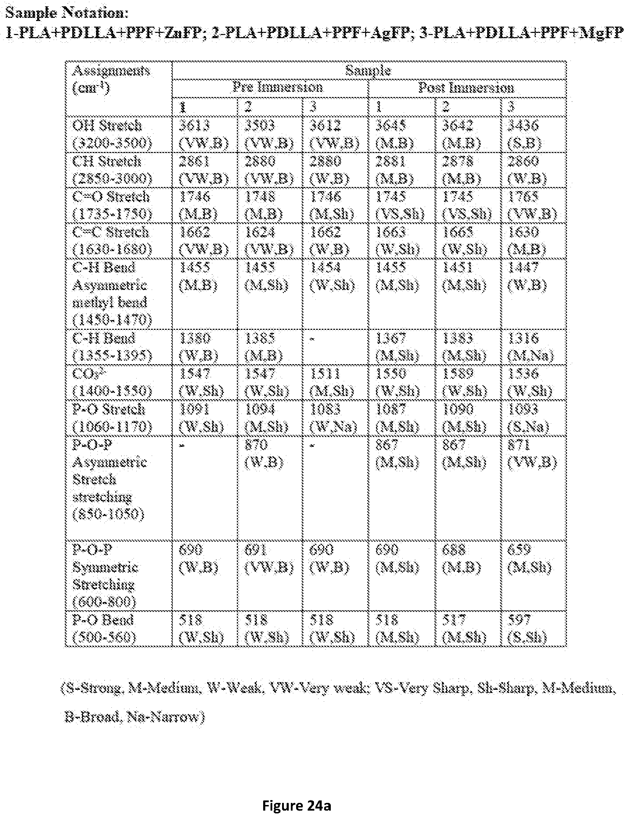

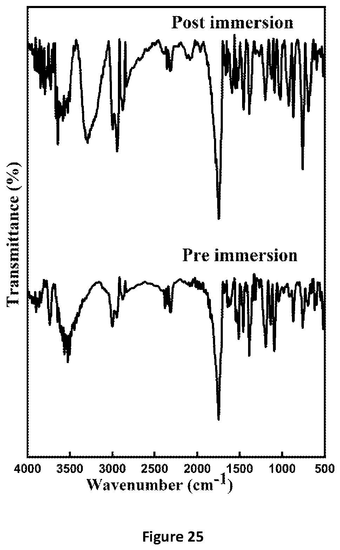

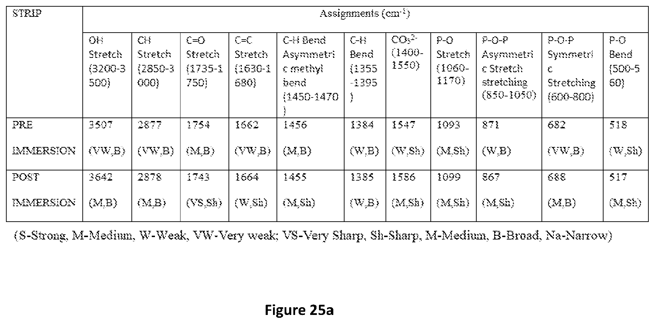

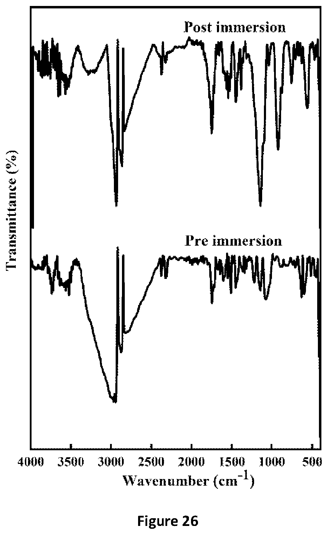

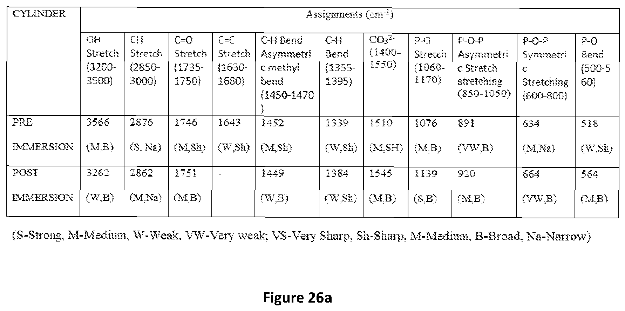

A Small amount of pre and post immersion specimen (pre and post immersion refers to the scaffolds before immersion in the SBF and after immersion in SBF and drying). were ground separately with potassium bromide and made into pellets. This pellets were used for the analysis. The analysis was done in the spectral range of 500-4000 $cm^{-1}$ by Fourier transform infrared-8400S spectrophotometer, Shimadzu, Japan. (Figure 21-26).

The predominant functional groups of the composite seen in the FTIR evauation by their respective spectral ranges are alcohol (3200-3500$cm^{-1}$),alkanes (2850-3000 $cm^{-1}$),saturated ketone(1735-1750$cm^{-1}$), alkenes (1630-1680$cm^{-1}$),asymmetric methyl bend (1450-1470 $cm^{-1}$) methyl bending(1350-1395 $cm^{-11}$).The presense of P-O bend (560-500$cm^{-1}$) bands indicates the formation of calcium phosphate(CaO-$P_2O_5$)layer. The carbonate group $(CO_3)^{2-}$ (1400-1550 $cm^{-1}$) bands show the crystalline nature of the HA layer. The bands are observed at above 3500cm-1 which corresponds to the OH group. After 21 days of soaking in SBF the strong intensity and frequency shift of the $(CO_3)^{2-}$,P-O-P stretch and P-O bend groups reveal the interaction of the composite and HAp precipitation. (Figure 21-26, 21a-26a).

The shoulder peak at 1450-1410$cm^{-1}$ coupled with the weaker peak at 870-875 $cm^{-1}$ corresponds to type B carbonate vibrations, whereas the vibration regions of type A carbonate are 1450-1410 $cm^{-1}$ coupled with a band at 880 $cm^{-1}$.The type A and B carbonate are indistinguishable in these scaffolds because of the ester peaks also lies on the same region.Both type A and B carbonates are present in these scaffolds and their intensities are maximum at three selected compression moulded scaffold composites (PLA+PDLLA+PPF+ZnFP,PLA+PDLLA+PPF+AgFP, PLA+PDLLA+PPF+MgFP.) For the same compositesthe corresponding peaks for HAp in rapid heating combined gel foam casting is higher than the compression moulded scaffolds.

Although the HAp precipitation was noted in all the fabricated scaffolds,the intensity of the carbonated group$(CO_3)^{2-}$and phosphatebased group (P-O-P asymmetric and symmetric stretch,P-O bend) was observed as very high in Gel foam casting under Rapid heating than those of the corresponding composites made by compresion moulding.(Figure 21a-26a)

4) SEM_EDAX

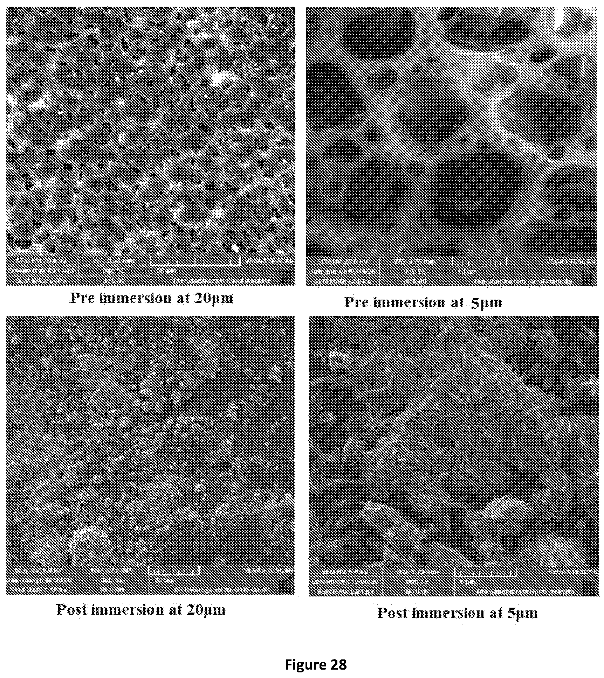

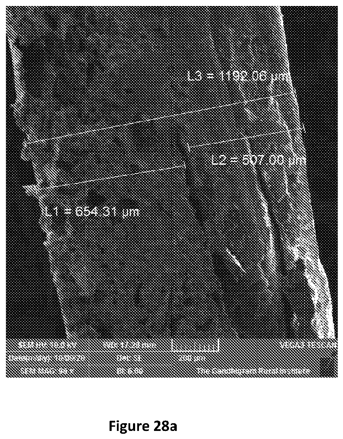

The morphological analysis and the semi-quantitative elemental concentration of the pre- and post-immersion samples prepared by compression moulding and pored by sugar leaching were examined using Scanning Electron Microscope - SEM (Model Ultra 55; Zeiss, Oberkochen, Germany) coupled with Energy Dispersive X-ray Spectrograph (ModelOxford Xmax50 EDS, Oxford Instrument, England) after gold sputtering. (Figure 28)

The specimens were cut into two halves to expose the interior of the scaffold. The exposed interior surface was sputtered with gold and analysed using the same Scanning Electron Microscope. (Figure 28a)

The width of crystallisation in relation to the actual width of the specimen was calculated as a percentage and has been tabulated to access the scaffold for Bio conversion. (Table 18).

Table 18

| Sample code | % of crystallinity |
|---|---|
| PLA+PPF+ZnFP | 87.31 |

(continued)

| Sample code | % of crystallinity |
|---|---|
| PLA+PDLLA+PPF+ZnFP | 76.19 |
| PLA+PPF+AgFP | 49.36 |
| PLA+PDLLA+PPF+AgFP | 72.7 |
| PLA+Diol+ZnFP | 59.54 |
| PLA+PDLLA+Diol+ZnFP | 78.89 |
| PLA+Diol+AgFP | 86.88 |
| PLA+PDLLA+Diol+AgFP | 97.42 |
| PLA+PPF+MgFP | 94.62 |
| PLA+PDLLA+PPF+MgFP | 82.09 |
| PLA+Diol+MgFP | 73.33 |
| PLA+PDLLA+Diol+MgFP | 78.68 |
| PLA+PDLLA+PPF+ZnFP(RH) | 73.62 |
| PLA+PDLLA+PPF+AgFP(RH) | 81.42 |
| PLA+PDLLA+PPF+MgFP(RH) | 87.34 |

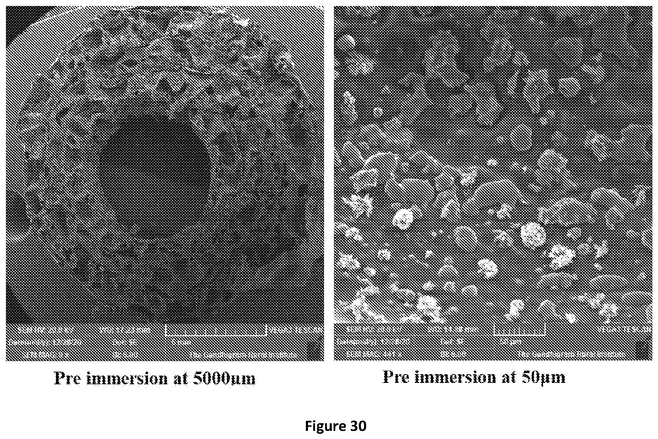

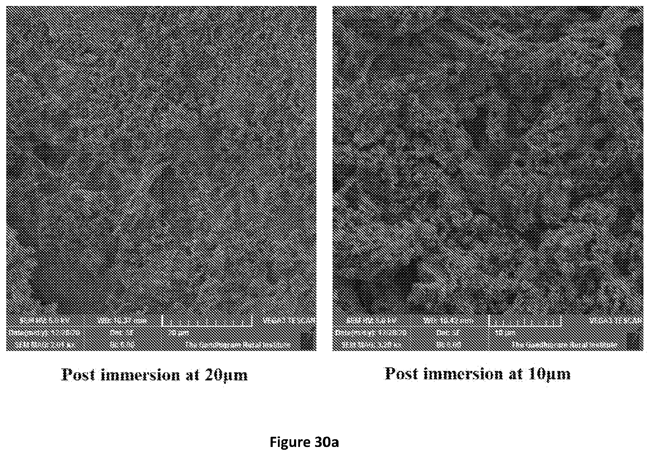

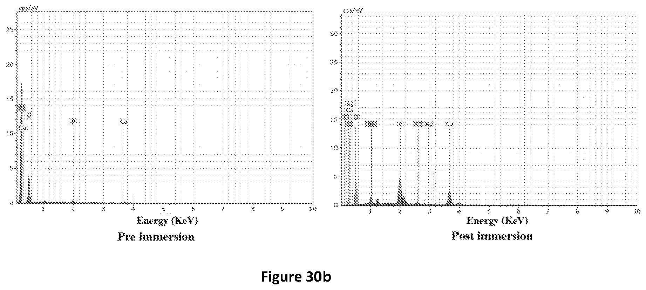

A single layer of the composite was made by Gel Foam Casting under Rapid Heating. A cylinder with a inner core diameter of 5mm was made with the composite. SEM evaluation of the single layer specimen made by Gel Foaming under Rapid heating and the multi layered cylinder were done after gold sputtering. (Model Ultra 55; Zeiss, Oberkochen, Germany) (Figure 30)

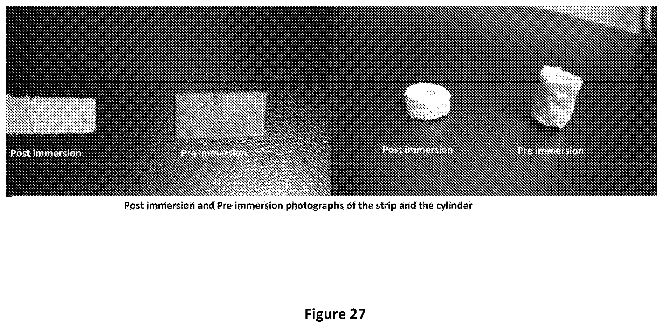

The similar specimens subjected to in vitro evaluation were analysed by the same way in the same Scanning Electron Microscope to assess the degree of surface pores and the change in crystallinity after in-vitro study (Figure 30a). The clinical photograph of a stirp of composite and a cylindrical composite ,both made by gelfoam casting under rapid heating shows the retention of the shape after SBF immersion for 21 days ,but the complete change in the colour and the texture indicating the crystalline conversion (Figure 27). The SEM of a compression moulded scaffold in two different magnification both before and after in vitro evaluation are shown in Figure 28 which shows very scarce amount of crystallisation in the pre invitro evaluation and the homogenous pores being well exhibited. After 21 days of immersion in SBF the crystalline conversion is well seen and all the pores have been near completely clogged with the crystals formed.

The specimens after SEM study were broken into two halves and the interior was evaluated by SEM for the crystallisation percentage of the thickness (Figure 28a).There was no significant change in the percentage and it can be infered all the composites has near equql conversion once the pores allow penetration of the SBF inside except the absence of PDLLA had some significance in the extent of crystalline conversion (Table 18).

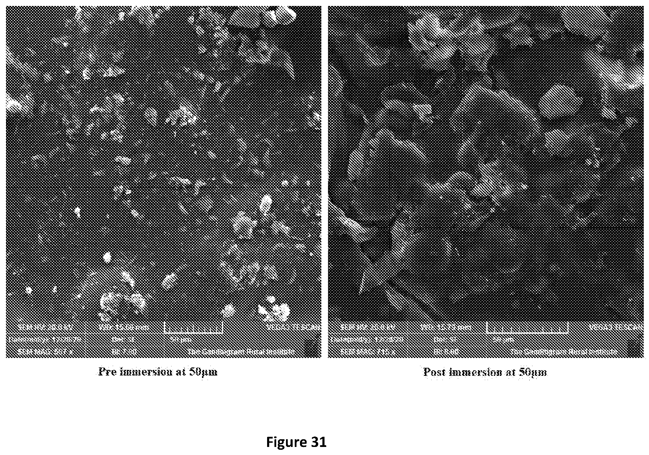

SEM study of a strip of scaffold made by gel foam casting under rapid heating in the pre in vitro status shows specks of crystallisation indicating the high hydrophilicity of the scaffold (Figure 31) and the post immersion evaluation of the same shows complete conversion into crystallinity which proves the high bioresorbability of the scaffold (Figure 31a)

The SEM evaluation of a cylindrical scaffold made by rapid heating under low magnification shows the adequacy of pores. This proves the homogenising of the polymers by the simple method adapted(Figure 30). The pre in-vitro and the post in-vitro SEM clearly shows the complete crstallisation that has occurred.(Figure 30a) The EDX evaluation of the pre and post in vitro SEM confirms the high level of carbonated hydroxy apatite formation in the scaffold (Figure 30 b).

5) MICRO -CT EVALUATION

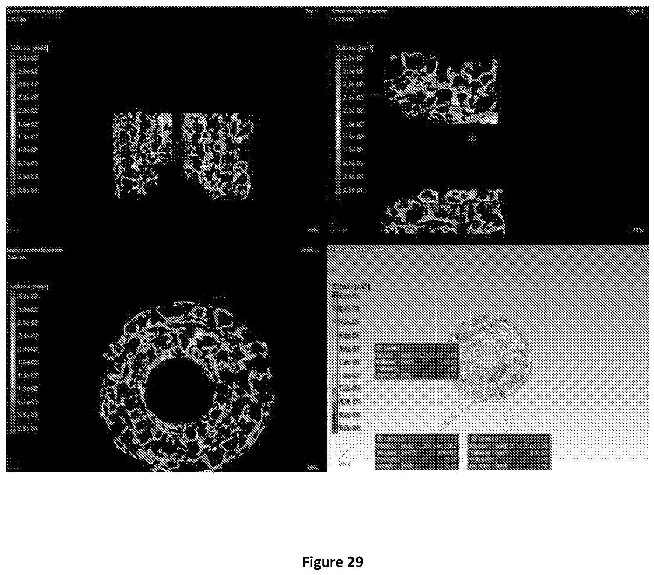

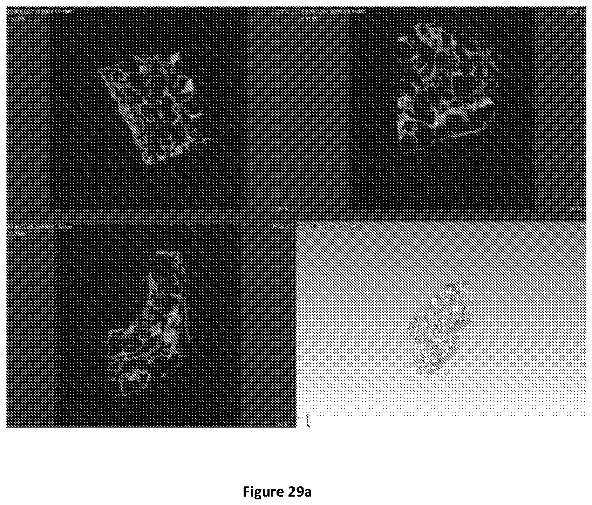

The surface and internal architecture of the scaffolds made in the single layer of strip, and the multi-layered cylinder were evaluated by the GE SRμCT analyser at various voxels and were 3D reconstructed. The porosity was assessed in all three planes (the axial, coronal and the sagittal). This disclosed the degree of porosity and the extent of the interporous connection. (Figure 29).

The specimens were subjected to in-vitro evaluation (immersed in SBF under 5% $CO_2$ environment at 37°C for 21 days) and the change was recorded by photograph (Figure 27) The post immersion Micro-CTevaluation showed complete crystallisation. (Figure 29a).

MICRO-CT evaluation of the cylindrical scaffold made by gel foam casting under rapid heating proved the

following factors a)The scaffold had no layering and was continuous.b)There was adequqtate porosity and the pore sizes were varying. c) Thepores were all well connected by interpores (Figure 29). The same specimen after in vitro evaluations had complete conversion to crystalline nature with the preservation of the deeper pores (Figure 29a).

**EXAMPLE 8: *In Vivo* evaluation of the composite granules:**

The in vivo studies were conducted with the Ethics committee approval (Ethical committee approval no **ABS/IAEC/18-10-2019/003-)**

A single species of Orictologus cuniculus was purchased from King Institute, Chennai, India and domesticated over a period of two weeks. The day night rhythm was maintained and was fed on good nourishing food. The adaptation was confirmed by the gain in weight of 150 g in two-week time. (1800- 1950g). The composite (PLA+PDLLA+PPF+AGFP) granules were prepared by morcellation of the scaffold and sterilised by ethylene oxide gas.

The animal was given a premedication of pedichloryl syrup (2.5 ml) thirty minutes before surgery. Intra muscular ketamine anaesthesia was given in the dose of 45 mgs per kilogram body weight and waited for ten minutes to get the full dissociated anaesthetic effect. The anaesthetic effect was maintained by oxygen and sevoprim inhalation through mask

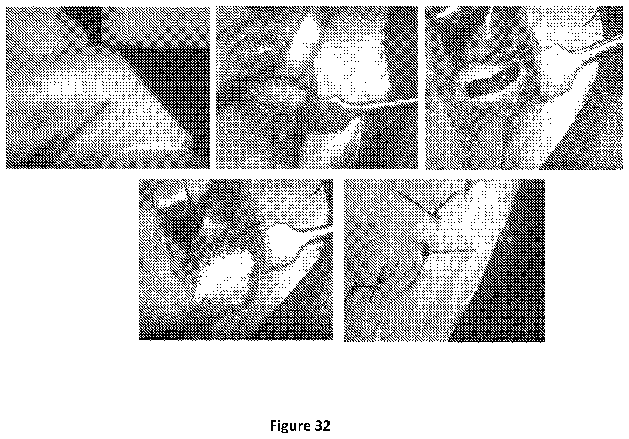

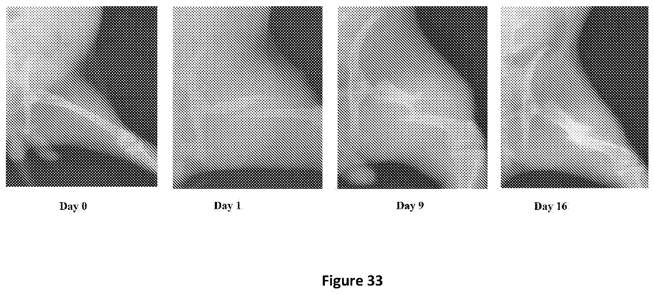

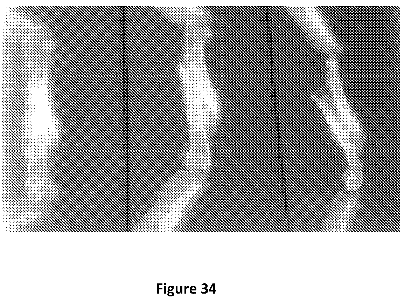

The left thigh was repeatedly painted with 10% povidone iodine and ethylene alcohol. Xylocaine 2% with adrenaline was injected in the line of incision as an additional analgesia and also a haemostatic agent. The skin incision made on the antero lateral aspect was rolled down to expose the posterior boarder of the quadriceps muscle. Using sharp dissection, the muscle was slit open and enlarged by thin bone spikes to expose the antero lateral aspect of the thigh bone. Using an electric dental burr of 1mm a trough was made for a length of 2cms.This exposed the medullary cavity. It was packed with the sterile composite powder. Liberal saline wash was given to wash off the spilled over composite materials. The spikes once removed the muscle fell back into position completely covering the bony trough. Two 3-0 vicryl stitches were used to close the muscle. The skin incision which was far away from the bone work was closed with 3-0 ethilon. A single dose of ceftrioxazone 250 mg was given intra muscularly. (Figure 32). It was found that the femur had fractured and the ends were apart (Figure 34). Neither immobilisation of the femur or any form of fixation was done. The rabbit was not limping and was feeding well. There was only a flare of the ends of the fracture and there was no evidence of any callus on the 9$^{th}$ post-operative day. After another week (Day 16) the limb when examined clinically and it had a sound union. X-ray taken showed abundant callus not only in the fracture end but all along the femur where the trough had been made and even below (Figure 33.)

The animal was euthanized, the limb harvested, skin and muscles were peeled off and an abundant amount of callus was found to have united the fracture very strongly. The dissected specimen was x-rayed and the specimen preserved in 10% formalin. (Figure 34)

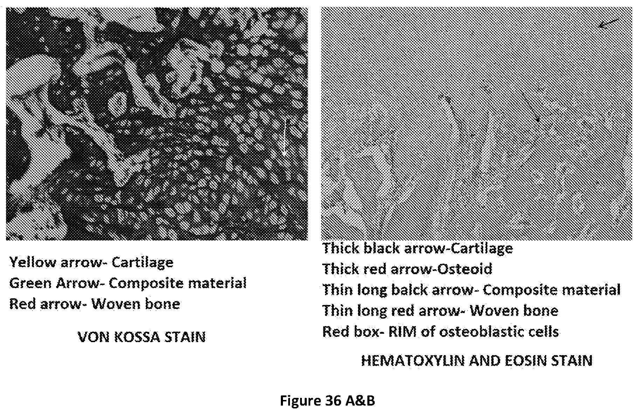

The specimen was prepared and the decalcified specimen was sectioned axially to exhibit the two segments of the femur with the intervening tissue formed. The specimen was stained using regular eosin-haematoxylin stain and also von kossa stain. (Figure 36).

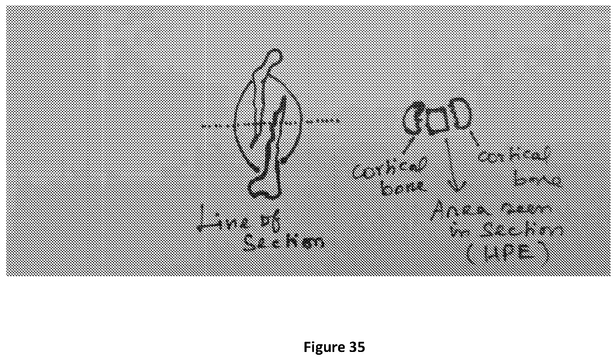

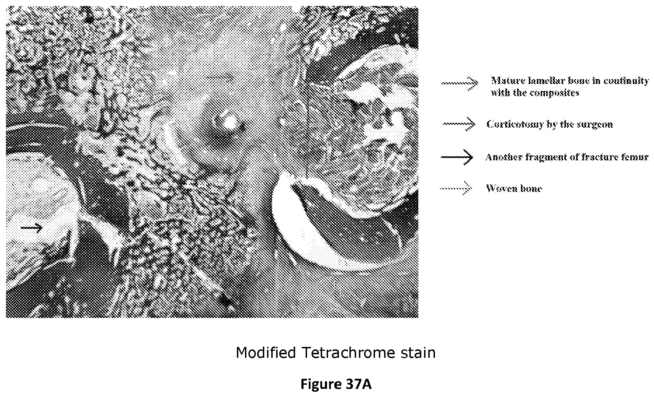

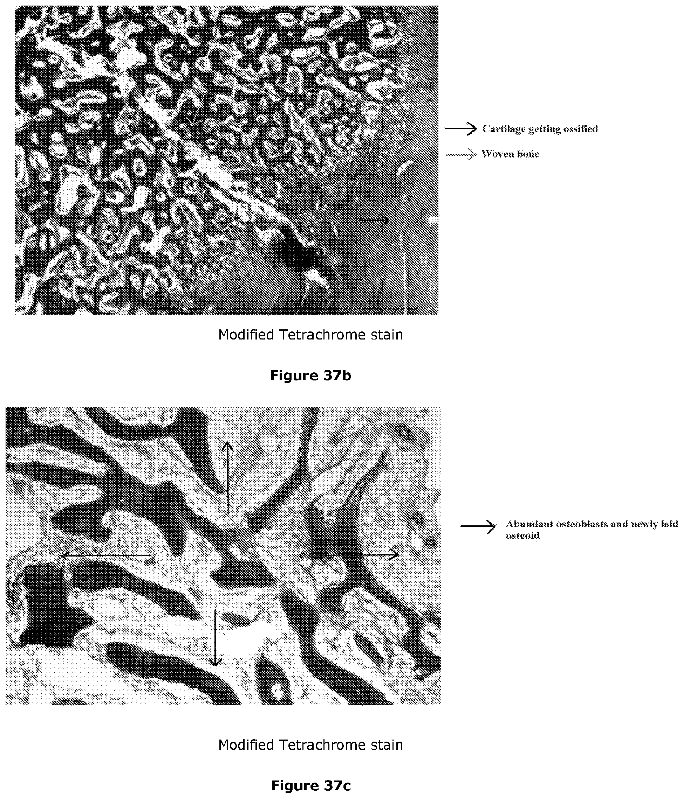

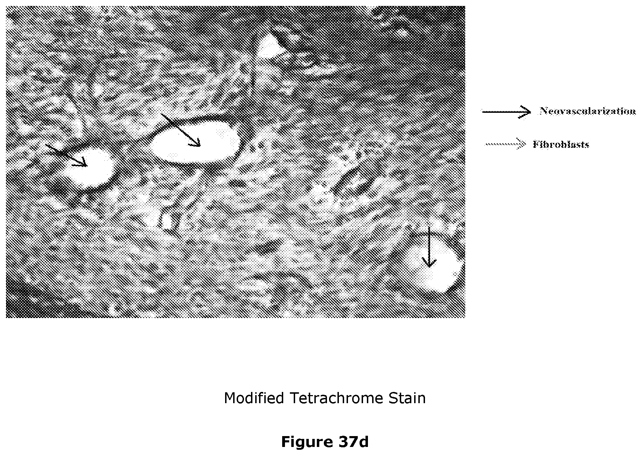

The procedure adapted has been serially shown in the photographs (Figure 32). Though the limb got fractuered it did not receive any specific treatment for it. Still the 9$^{th}$ day there was a scarce respose to heal but by the 16$^{th}$ day it had soundly united.(Figure 33). The xray of the specimen after dissection showed the extension of the callus almost over the entire femur .(Figure 34). The HPE was specifically focused towards the tissue between the fractured ends where the granules had been packed (Figure 35). The significant observations were a)Nearly the whole of the granules had resorbed except occasional trace of it. b)abundant cartilage had formed between the ends indicating the enchondral ossification. c) woven bone formed inbetween the ends of the fracture was a proof of the rapidity of the fusion occuring d)the absence of multinucleated giant cells indicate the bio compatability of the composite.e)similar features were observed in both the staining(Figure 36). The modified tetrachrome staining throws much more information than the above two. a)The new lamellar bone formed in continuity with the resorbing composite granule b)the sound union by the woven bone formed from chondral ossification c)the abundance of osteoblasts and the osteoid d)exuberent neo vascularisation among the fibroblasts are well seen (Figure 37-a-b-c-d)

**EXAMPLE 9: *In Vivo* evaluation of the composite strips**

The in vivo studies were conducted with the Ethics committee approval (Ethical committee approval no **ABS/IAEC/18-10-2019/003-)**

Three male rabbits were procured and domesticated in the same way as explained before. AgFP/ZnFP/MgFP composites were made with PLA+PDLLA+PPF by Gelfoam casting under rapid heating. They were of 1mm thickness and cut into size of 2*20mm. The cut specimens were sterilised by Ethylene oxide gas sterilisation.

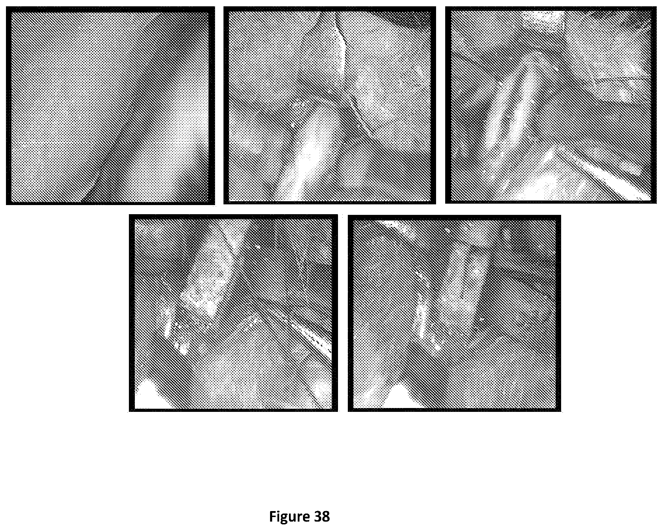

The animals were anaesthetised, limb prepared and femur exposed as described in the previous study. Narrow cuts were made with no701 conical dental burr at an angle of 45° to the femur to make it extremely thin cut .3-0 vicryl was threaded around the femur and both the ends were kept free. Two layers of the 2*20mm sterilised composite was kept over

the cut made allowing the marrow blood to choke the specimen. The vicryl was tied around the specimen so that the specimen does not slip or move away and the wound was closed in layers (Figure 38). The procedure was done for all the three specimens one on each animal.

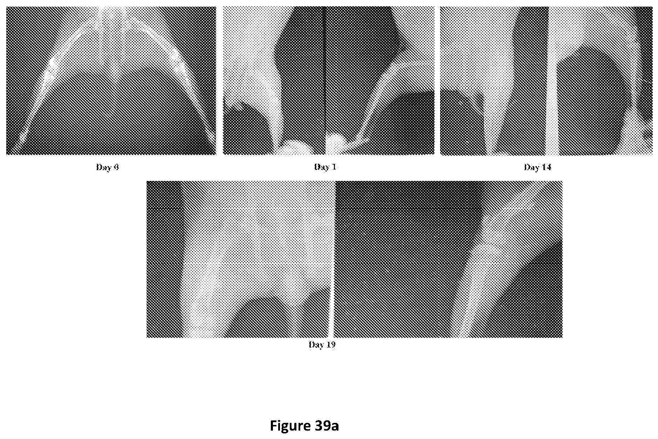

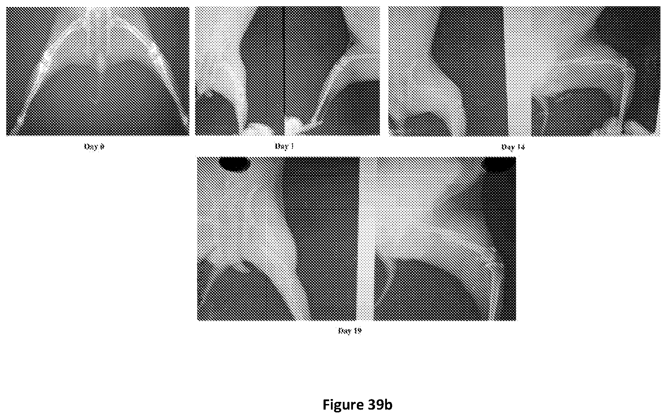

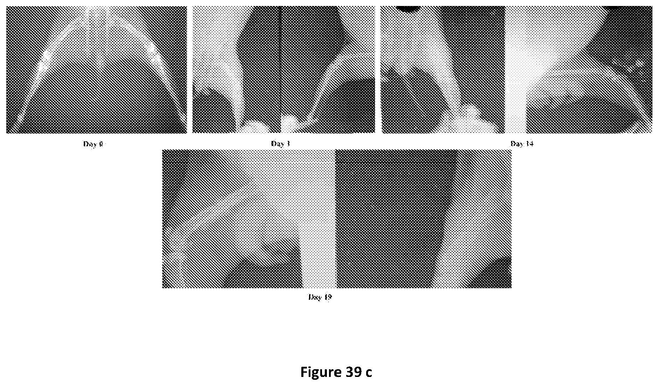

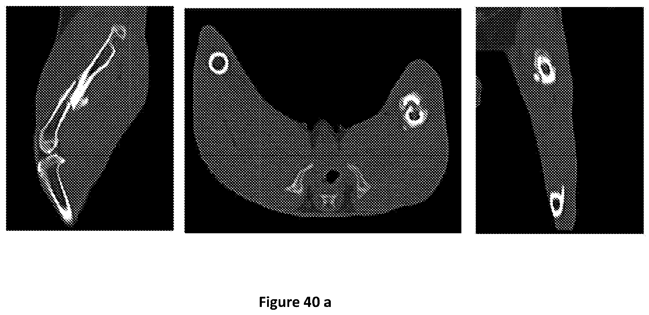

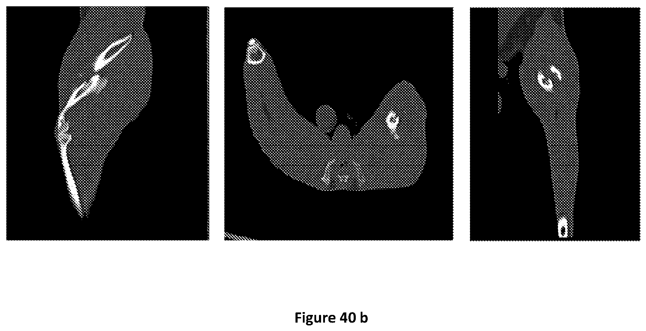

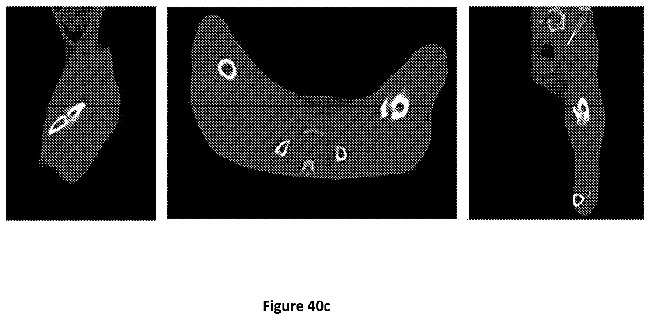

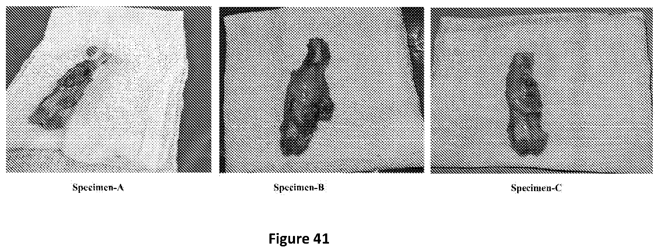

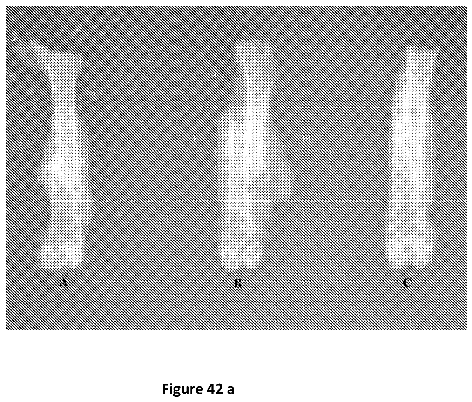

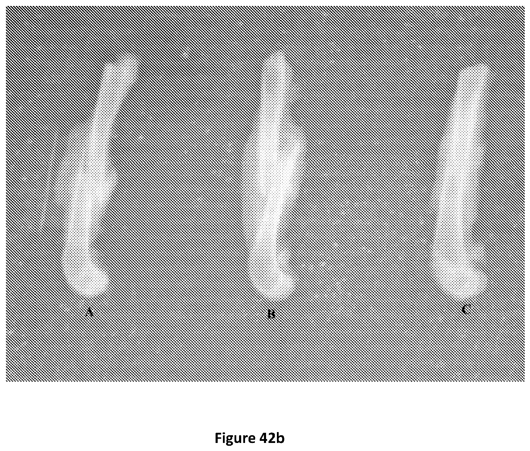

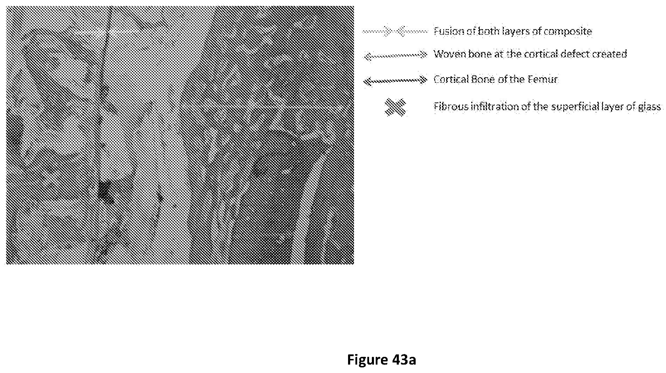

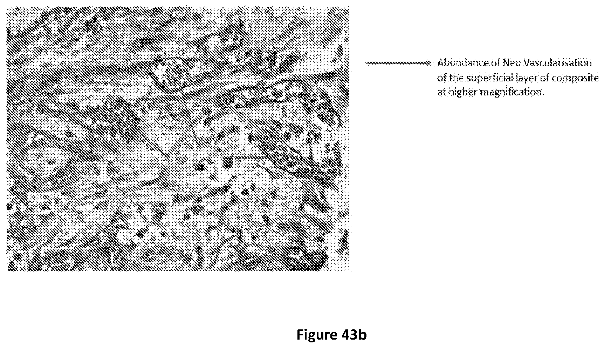

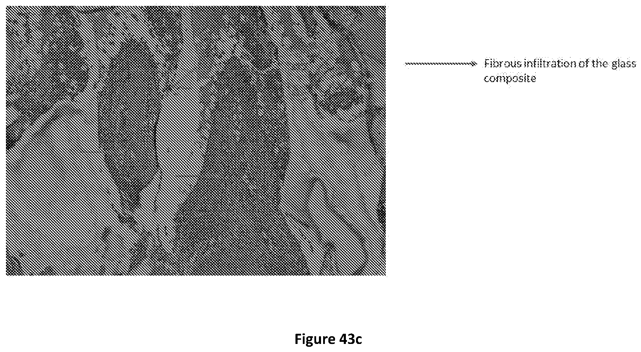

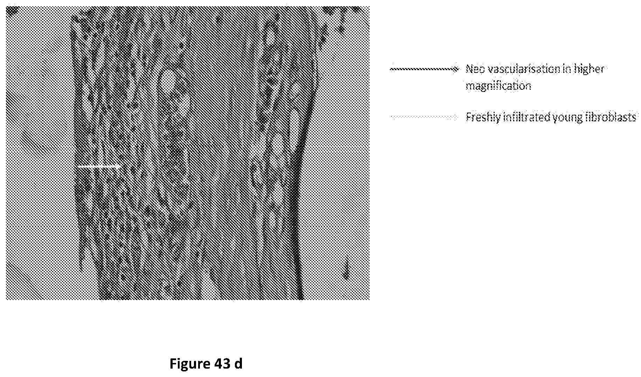

The animals were cared for in the post-operative period with nourishing food. The day one x-ray didn't show the specimen in either view as the specimens were translucent to the x-ray. X-ray evaluation was done under sedation on the 1st,9th and 16th day. (Figure 39a,39b,39c) Clinical union occurred as early as the 15th day. CT evaluation was done on the 19th day. (Figure 40, 40a, 40b) The animals were euthanized as per the protocol and the limb harvested, denuded of skin and muscles and bone preserved in 10% formalin (Figure 41). X-Rays of the specimens taken (Figure 42,42a) and then sent for histo pathological evaluation in both EH stain and Eosin stain (Figure 43,a,b,c,d.)

The procedure adapted is shown in the serial photographs in the Figure 38,where two layers of 1mm thick strips have been placed over a very narrow corticotomy wound in the shaft of femur and has been retained in position by a single 3-0 vicryl encircling knot. Figure 39 a, b, c, shows no evidence of the placed composite sheet or the corticotomy made as the composite is not radio opaque and the furrow is very narrow. But the x-rays taken on the 9th day showed all three animals had fractured their femur.No specific treatment like immobilisation or interference was done for the fracture.Clical union occurred as early as 15th day, and was confirmed by x-ray on 16th day and CT scan on 19th day.(Figure 40 a,b,c).The harvested limb after euthanizing the animal showed the composite strip was adherent to the bone underneath (Figure 41). The X-ray of the specimens showed abundant callus along the fracture (Figure 42)and the composite strip was not seen in the X-ray.

The histo pathalogical evaluation showed the following features (Figure 43 a) a)Both the layers of the scaffold had merged into one layer b)The composite had attached to the bone beneath. c)There was abundunt woven bone formed beneath the composite strip at the level of the corticotomy. (Figure 43 b) d)The second layer of the composite srip kept away from the corticotomy had profuse infiltration of fibrocytes. (Figure 43 c,d) e)The fibrous changeover in the superficial layer of the composite had abundant neovascularisation These changes confirm the osteo induction potential of the composite,the ability of the composite to go for bioconversionand high bioactivity of the composite.

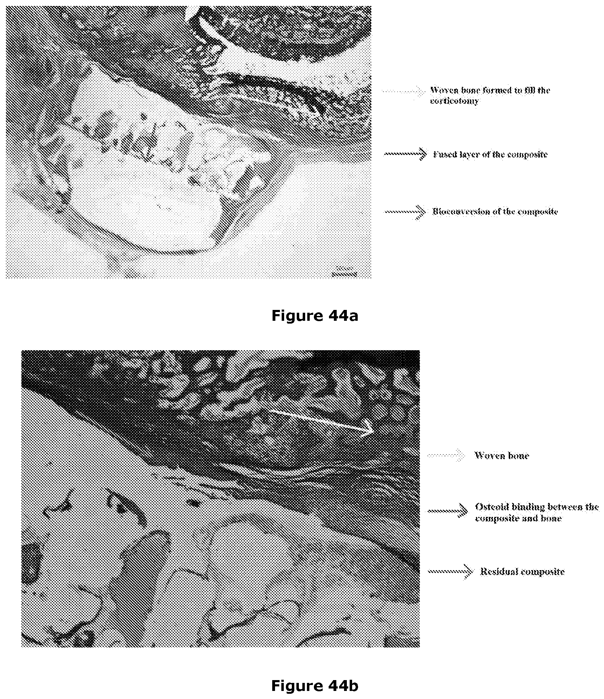

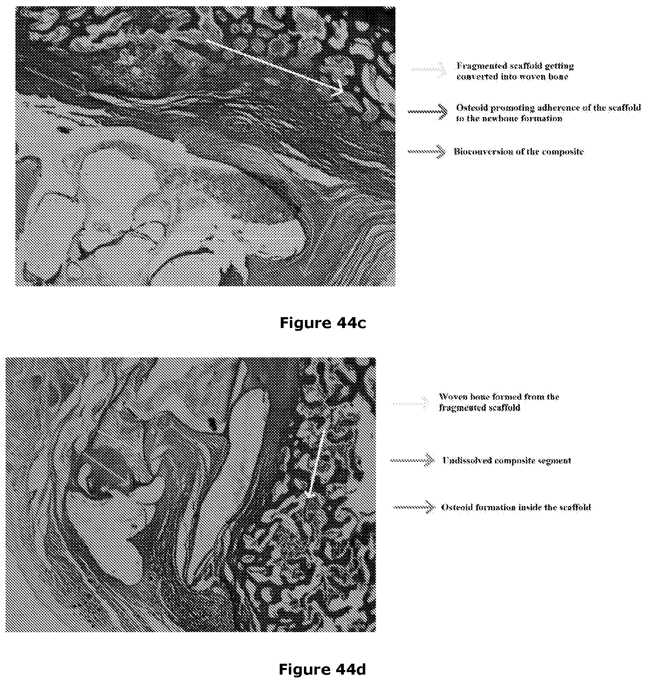

The modified Tetrachrome staining of the specimens with the cross section at the level of the composite confirmed the findings by EH stain and showed the additional features. Fig 44a shows conversion of the fragmented composite forming woven bone to heal the corticotomy made and the binding of the two layers of the composite strip and random infiltration of the layer close to the bone with fibroblasts and specks of osteiod. On Higher magnification ( 44b) the fusion of the composite strip to the underlying bone by osteoid is well seen .On further magnification (44 c)the infiltration of the composite by newly formed layers of osteoid are well made out replacing the dissolved area of the composite. Fig 44d shows the adhesion of the composite strip, the composite strip dissolving and disintegerating to form new woven bone healing the corticotomy , the phenomenal laying of new osteoid in the dissolved portion of the composite.

**EXAMPLE 10: Preparation of the scaffold by 3 D printing**

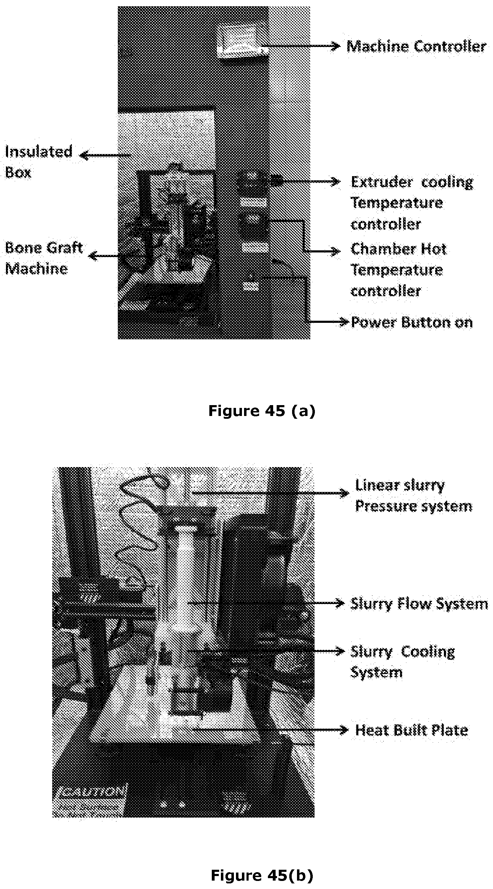

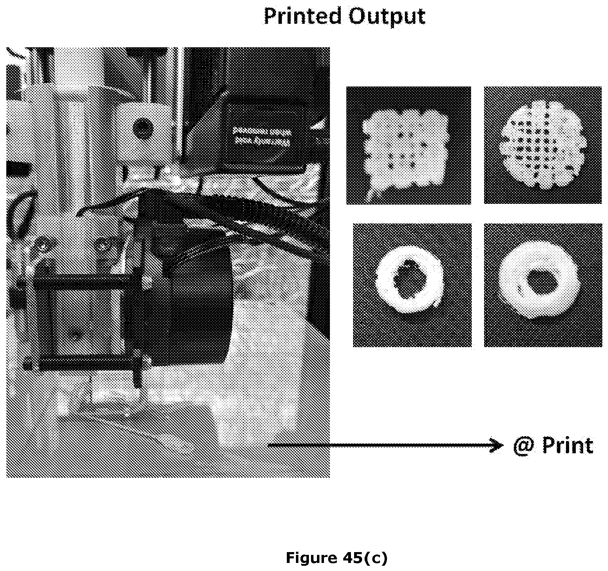

Fused filament fabrication (FFF) 3D printer is generally used for fusing plastics, extruded at a higher temperature and cooled to room temperature to build the 3 D model into a product. A customised 3D printer was manufactured for fabricating the composite. The ink printer is maintained at a cool temperature in the printer so that the homogeneity obtained between the components of the composite is not lost. (Figure 45b). For that purpose, a special cooling chamber was designed. It cools the slurry extruder at 15-20$^0$ C. The slurry is extruded to a plate built to get heated upto 100°C and the chamber temperature of 30-40$^0$C is maintained (figure 45a). The extrusion was controlled by conventional CAD software and the required designs were printed (Figure 45c).

**ADVANTAGES:**

The composite can be made as granules or powders or their mixture which can be used as a filler for bone voids arising out of lesions, infections, tumours of bone which will get converted to bone in a shorter period avoiding amputations and also reduce the morbidity by reducing the time taken for bioconversion.

The composite made as strips can be used as an only graft like that of a Phemister graft which is the commonest type of autogenous graft used by the orthopaedic surgeon. This will reduce the morbidity of the surgery and avoid a second incision to harvest the autograft.

The composites as cylindrical grafts can be used as an interposition graft and can save many long bones with critical sized defects arising out of trauma or other lesions.

The composites can be custom made to a graft by rapid prototyping method so that a specific portion of a bone can be replaced when diseased rather than being amputated.

The synthetic composite of the invention has following characters

a) Biocompatible; b) Bioactive;c) Biodegradable; d) Nontoxic to the recipient;e) Bioconductive; f) Bioinductive; g) Bioconvertible; h) Rate of degradation to match the rate of bio-conversion; i) sterilisable; j) easy to be produced in bulk;

k) workable to the desired shape; l) cost effective.

## Claims

1. A synthetic composite for a bone graft comprising:

   bio inert polymers comprising polylactic acid, poly D, L-Lactic acid;
   bio active polymer consisting of polypropylene fumarate, or a diester of fumaric acid and propylene diol; and
   a bioactive inorganic component consisting of a metal fluorophosphates glass powder wherein the bioactive inorganic metal fluorophosphates glass powder is upto 30% (w/w) of the composite.

2. The composite as claimed in claim 1, wherein the metal fluorophosphate glass is selected from one of zinc fluorophosphate, magnesium fluorophosphate, or silver fluorophosphate.

3. The composite as claimed in claim 1, wherein the polylactic acid is in the range of 54% (w/w) to 68% (w/w).

4. The composite as claimed in claim 1, wherein the diester of fumaric acid and propylene diol is in the range of 3% (w/w) to 10% (w/w).

5. The composite as claimed in claim 1, wherein the poly D, L-lactic acid is in the range of 10% (w/w) to 28% (w/w).

6. The composite as claimed in claim 1, wherein the polypropylene fumarate is in the range of 3% (w/w) to 10% (w/w).

7. The composite as claimed in claim 1, wherein the metal fluorophosphate in the composite is in the range of 10% (w/w) to 30% (w/w).

8. The composite as claimed in claim 1, comprising

   zinc fluorophosphate,
   a bio inert polymer; and
   bio active polymer consisting of polypropylene fumarate, or diester of fumaric acid and propylene diol;

   wherein the bio inert polymer is polylactic acid or a combination of polylactic acid and poly D, L-Lactic acid.

9. The composite as claimed in claim 1, comprising:

   magnesium fluorophosphate,
   a bio inert polymer; and
   bio active polymer consisting of polypropylene fumarate, or diester of fumaric acid and propylene diol;

   wherein the bio inert polymer is polylactic acid or a combination of polylactic acid and poly D, L-Lactic acid.

10. The composite as claimed in claim 1, comprising:

    silver fluorophosphate,
    a bio inert polymer; and
    bio active polymer consisting of polypropylene fumarate, or diester of fumaric acid and propylene diol;

    wherein the bio inert polymer is polylactic acid or a combination of polylactic acid and poly D, L-Lactic acid.

11. The composite as claimed in claim 1, is a powder, or a scaffold; optionally wherein the scaffold is a strip or a cylinder or a tube and the like.

12. A method of preparing the synthetic composite as claimed in claim 11, comprising the steps of:

    - mixing the composite as claimed in claim 1 in a solvent with the magnetic stirrer or by sonicating to obtain a homogenous mixture;

- casting the mixture over hot glass plate and bringing the solution to boil;
- evaporation of the solvent by continuous boiling; and
- obtaining an interconnected porous scaffold with the homogenous distribution of the components of the composite.

**13.** The method as claimed in claim 12, wherein the solvent is selected from dichloromethane, acetone, toluene and chloroform.

**14.** The method as claimed in claim 12, wherein the porosity of the scaffold ranges from 20%-40%.

**15.** The composite as claimed in claim 11, wherein the scaffold is made by a custom made 3D printer by direct ink printing technique comprising the following steps:

- homogenisation of the components and cooling the same to 10 to 30 °C;
- loading of the ink into a pressure-controlled extruder which is non adhesive to the ink;
- programming of the shape, thickness, porosity, and layers required which is fed by a computer;
- obtaining the desired scaffold.

## Patentansprüche

**1.** Synthetischer Verbundstoff für ein Knochentransplantat, umfassend:

bioinerte Polymere, umfassend Polymilchsäure, Poly-D, L-Milchsäure;
bioaktives Polymer, bestehend aus Polypropylenfumarat oder einem Diester aus Fumarsäure und Propylendiol; und
eine bioaktive anorganische Komponente, bestehend aus einem Metallfluorphosphat-Glaspulver, wobei das bioaktive anorganische Metallfluorphosphat-Glaspulver bis zu 30 % Massenanteil des Verbundstoffs ist.

**2.** Verbundstoff nach Anspruch 1, wobei das Metallfluorphosphatglas ausgewählt ist aus Zinkfluorphosphat, Magnesiumfluorphosphat oder Silberfluorphosphat.

**3.** Verbundstoff nach Anspruch 1, wobei die Polymilchsäure in dem Bereich von 54 % Massenanteil bis 68 % Massenanteil ist.

**4.** Verbundstoff nach Anspruch 1, wobei der Diester aus Fumarsäure und Propylendiol in dem Bereich von 3 % Massenanteil bis 10 % Massenanteil ist.

**5.** Verbundstoff nach Anspruch 1, wobei die Poly-D, L-Milchsäure in dem Bereich von 10 % Massenanteil bis 28 % Massenanteil ist.

**6.** Verbundstoff nach Anspruch 1, wobei das Polypropylenfumarat in dem Bereich von 3 % Massenanteil bis 10 % Massenanteil ist.

**7.** Verbundstoff nach Anspruch 1, wobei das Metallfluorphosphat in dem Verbundstoff in dem Bereich von 10 % Massenanteil bis 30 % Massenanteil ist.

**8.** Verbundstoff nach Anspruch 1, umfassend:

Zinkfluorphosphat,
ein bioinertes Polymer; und
bioaktives Polymer, bestehend aus Polypropylenfumarat oder Diester aus Fumarsäure und Propylendiol;
wobei das bioinerte Polymer Polymilchsäure oder eine Kombination aus Polymilchsäure und Poly-D, L-Milchsäure ist.

**9.** Verbundstoff nach Anspruch 1, umfassend:

Magnesiumfluorphosphat,

ein bioinertes Polymer; und
bioaktives Polymer, bestehend aus Polypropylenfumarat oder Diester aus Fumarsäure und Propylendiol;
wobei das bioinerte Polymer Polymilchsäure oder eine Kombination aus Polymilchsäure und Poly-D, L-Milchsäure ist.

**10.** Verbundstoff nach Anspruch 1, umfassend:

Silberfluorphosphat,
ein bioinertes Polymer; und
bioaktives Polymer, bestehend aus Polypropylenfumarat oder Diester aus Fumarsäure und Propylendiol;
wobei das bioinerte Polymer Polymilchsäure oder eine Kombination aus Polymilchsäure und Poly-D, L-Milchsäure ist.

**11.** Verbundstoff nach Anspruch 1, der ein Pulver oder ein Gerüst ist; optional wobei das Gerüst ein Streifen oder ein Zylinder oder ein Rohr und dergleichen ist.

**12.** Verfahren zum Herstellen des synthetischen Verbundstoffs nach Anspruch 11, umfassend die folgenden Schritte:

- Mischen des Verbundstoffs nach Anspruch 1 in einem Lösungsmittel mit dem Magnetrührer oder durch Beschallung, um ein homogenes Gemisch zu erlangen;
- Gießen des Gemischs auf eine heiße Glasplatte und Bringen der Lösung zum Sieden;
- Verdampfen des Lösungsmittels durch kontinuierliches Sieden; und
- Erlangen eines miteinander verbundenen porösen Gerüsts mit der homogenen Verteilung der Komponenten des Verbundstoffs.

**13.** Verfahren nach Anspruch 12, wobei das Lösungsmittel ausgewählt ist aus Dichlormethan, Aceton, Toluol und Chloroform.

**14.** Verfahren nach Anspruch 12, wobei die Porosität des Gerüsts von 20 % bis 40 % reicht.

**15.** Verbundstoff nach Anspruch 11, wobei das Gerüst durch einen maßgefertigten 3D-Drucker mittels direkter Tintendrucktechnik gefertigt wird, umfassend die folgenden Schritte:

- Homogenisieren der Komponenten und deren Abkühlen auf 10 bis 30 °C;
- Laden der Tinte in einen druckgesteuerten Extruder, der für die Tinte nicht haftend ist;
- Programmieren der erforderlichen Form, Stärke, Porosität und Schichten, die von einem Computer eingespeist wird;
- Erlangen des gewünschten Gerüsts.

## Revendications

**1.** Composite synthétique pour une greffe osseuse, comprenant :

des polymères bio-inertes comprenant de l'acide polylactique, de l'acide poly-D,L-lactique ;
un polymère bioactif consistant en du poly(fumarate de propylène), ou un diester d'acide fumarique et de propylène glycol ; et
un composant inorganique bioactif consistant en une poudre de verre de fluorophosphate métallique,

dans lequel la poudre de verre de fluorophosphate métallique inorganique bioactive représente jusqu'à 30 % (p/p) du composite.

**2.** Composite selon la revendication 1, dans lequel le verre de fluorophosphate métallique est choisi parmi le fluorophosphate de zinc, le fluorophosphate de magnésium ou le fluorophosphate d'argent.

**3.** Composite selon la revendication 1, dans lequel l'acide polylactique se situe dans la plage de 54 % (p/p) à 68 % (p/p).

**4.** Composite selon la revendication 1, dans lequel le diester d'acide fumarique et de propylène glycol se situe dans la

plage de 3 % (p/p) à 10 % (p/p).

**5.** Composite selon la revendication 1, dans lequel l'acide poly-D,L-lactique se situe dans la plage de 10 % (p/p) à 28 % (p/p).

**6.** Composite selon la revendication 1, dans lequel le poly(fumarate de propylène) se situe dans la plage de 3 % (p/p) à 10 % (p/p).

**7.** Composite selon la revendication 1, dans lequel le fluorophosphate métallique dans le composite se situe dans la plage de 10 % (p/p) à 30 % (p/p).

**8.** Composite selon la revendication 1, comprenant

du fluorophosphate de zinc,
un polymère bio-inerte ; et
un polymère bioactif consistant en du poly(fumarate de propylène), ou un diester d'acide fumarique et de propylène glycol ;
dans lequel le polymère bio-inerte est l'acide polylactique ou une combinaison d'acide polylactique et d'acide poly-D,L-lactique.

**9.** Composite selon la revendication 1, comprenant :

du fluorophosphate de magnésium,
un polymère bio-inerte ; et
un polymère bioactif consistant en du poly(fumarate de propylène), ou un diester d'acide fumarique et de propylène glycol ;

dans lequel le polymère bio-inerte est l'acide polylactique ou une combinaison d'acide polylactique et d'acide poly-D,L-lactique.

**10.** Composite selon la revendication 1, comprenant :

du fluorophosphate d'argent,
un polymère bio-inerte ; et
un polymère bioactif consistant en du poly(fumarate de propylène), ou un diester d'acide fumarique et de propylène glycol ;

dans lequel le polymère bio-inerte est l'acide polylactique ou une combinaison d'acide polylactique et d'acide poly-D,L-lactique.

**11.** Composite selon la revendication 1, qui est une poudre ou un support ; facultativement, dans lequel le support est une bande ou un cylindre ou un tube et similaires.

**12.** Procédé de préparation du composite synthétique selon la revendication 11, comprenant les étapes consistant à :

- mélanger le composite selon la revendication 1 dans un solvant avec un agitateur magnétique ou par sonication afin d'obtenir un mélange homogène ;
- couler le mélange sur une plaque de verre chaude et porter la solution à ébullition ;
- évaporer le solvant par ébullition continue ; et
- obtenir un support poreux interconnecté avec une distribution homogène des composants du composite.

**13.** Procédé selon la revendication 12, dans lequel le solvant est choisi parmi le dichlorométhane, l'acétone, le toluène et le chloroforme.

**14.** Procédé selon la revendication 12, dans lequel la porosité du support va de 20 % à 40 %.

**15.** Composite selon la revendication 11, dans lequel le support est réalisé au moyen d'une imprimante 3D sur mesure par une technique d'impression directe d'encre comprenant les étapes suivantes :

- homogénéisation des composants et refroidissement de ceux-ci à une température de 10 à 30 °C ;
- chargement de l'encre dans une extrudeuse à pression contrôlée qui est non adhésive vis-à-vis de l'encre ;
- programmation de la forme, de l'épaisseur, de la porosité et des couches requises, laquelle programmation est alimentée par un ordinateur ;
- obtention du support souhaité.

ALP Assay
ALP concentration (ng/ml)
180
160
140
120
100
80
60
40
20
0
FP
MgFP
ZnFP
TiFP
ZrFP
AgFP
SrFP
Control
100 µg
10 µg
1 µg
0.1 µg
Control

Figure 1

Fp

**Figure 2a**

MgFp

**Figure 2b**

**Figure 2c**

TiFp

**Figure 2d**

ZrFP

AgFp

Figure 2e

Figure 2f

SrFp

Figure 2g

Transmittance (%)
PPF
4000
3500
3000
2500
2000
1500
1000
500
Wavenumber (cm-1)

Figure 3

Heat flow (W g-1)
PPF
Temperature ( °C)

Figure 4

Mass (%)
Temperature ( °C)

Figure 4a

Diol
4000
3500
3000
2500
2000
1500
1000
500
Wavenumber (cm-1)

Figure 5

| SAMPLE | Assignments (cm$^{-1}$) | | | | | |
|---|---|---|---|---|---|---|
| | OH Stretch (3200-3500) | CH Stretch (2850-3000) | C=O Stretch (1735-1750) | C=C Stretch (1630-1680) | C-H Bend Asymmetric methyl bend (1450-1470) | C-H Bend (1355-1395) |
| PPF | 3160 | 2981 | 1747 | 1643 | 1457 | 1373 |
| 1,2 Diol | 3461 | 2980 | 1736 | 1640 | 1460 | 1378 |

Figure 5a

100
80
60
40
20
Mass (%)
0
100
200
300
400
500
600
Temperature (°C)

Figure 6

DSC Thermal Analysis
CHARACTERISATION OF PLLA
IV(DL) 3.3-4.3
ASH < 0.1%
RESIDUAL MONOMERS < 0.1%
RESIDUAL SOLVENT <890 ppm
TIN CONTENT < 60 ppm
Tg 53-60˚
MELTING >90˚
50.00
100.00
150.00
200.00
0.00
-2.00
-4.00
-6.00
-8.00

Figure 7

DSC Thermal Analysis
DSC
mW
0.00
-1.00
-2.00
20.00
40.00
60.00
80.00
100.00
Temp [C]
CHARACTERISATION OF PDLLA
IV(DL/G) 0.85-1.15
MW 100000-180000
Tg 50-60
PARTICULATE SIZE >500μm
CONTAMINATION 0%
File Name:
Detector Type: DSC-60A
Acquire Date:
Acquire Time:
Lot No:
Sample Name: PDLLA
Sample Weight:


Figure 8

Figure 9

Figure 10

Figure 11

SaOS2 Cells- Control
PLA+PDLLA
PLA+PDLLA+PPF
PLA+PDLLA+PPF+TiFP

Figure 12

PLA+PDLLA
PLA+PDLLA (Pored)
PLA+PDLLA+PPF
PLA+PDLLA+XPPF
PLA+PDLLA+PPF (Pored)
PLA+PDLLA+XPPF (Pored)
PLA+PDLLA+ZnFP
PLA+PDLLA+ZnFP (Pored)
PLA+PDLLA+PPF+ZnFP
PLA+PDLLA+PPF+ZnFP (Pored)
PLA+PDLLA+XPPF+ZnFP
PLA+PDLLA+XPPF+ZnFP (Pored)
Control

Figure 13

A
B
C
D

A-Salt leaching; B-Gel Pressing; C-Gas Foaming; D-Precipitation-Freeze drying

Figure 14

Figure 14a

Figure 14b

Cytotoxicity (MTT) Assay - Endothelial cells
Scaffold model
Cell viability %
120
100
80
60
40
20
0
1
2
3
4
5
6
7
8
9
10
11
12
CN
Groups

1-PLA+PDLLA+PPF+AgFP; 2-PLA+PPF+AgFP; 3-PLA+PDLLA+Diol+AgFP; 4-PLA+Diol+AgFP; 5-PLA+PDLLA+PPF+ZnFP; 6-PLA+PPF+ZnFP; 7-PLA+PDLLA+Diol+ZnFP; 8-PLA+Diol+ZnFP; 9-PLA+PPF+MgFP; 10-PLA+PDLLA+PPF+MgFP; 11-PLA+Diol+MgFP; 12-PLA+PDLLA+Diol+MgFP; CN-Control

Figure 15

Gro ups
1
2
3
4
5
6
24 hrs
48 hrs
Gro ups
7
8
9
10
11
12
24 hrs
48 hrs
Control 24 hrs
Control 48 hrs

Figure 15a

PA-Control-PLA+PPF+AgFP; DA-Control-PLA+Diol+AgFP; PA-1-PLA+PDLLA+PPF+AgFP; PZ-1 PLA+PDLLA+PPF+ZnFP; PZ Control-PLA+PPF+ZnFP; DZ Control-PLA+Diol+ZnFP; DA-1-PLA+PDLLA+Diol+AgFP; DZ-1-PLA+PDLLA+Diol+ZnFP; GAPDH-Control

Figure 16

PA-Control-PLA+PPF+AgFP; DA-Control-PLA+Diol+AgFP; PA-1-PLA+PDLLA+PPF+AgFP; PZ-1 PLA+PDLLA+PPF+ZnFP; PZ Control-PLA+PPF+ZnFP; DZ Control-PLA+Diol+ZnFP; DA-1-PLA+PDLLA+Diol+AgFP; DZ-1-PLA+PDLLA+Diol+ZnFP; GAPDH-Control

Figure 17

ABS-09 Osteocalcin
ABS-10 Osteocalcin
ABS-11 Osteocalcin
ABS-12 Osteocalcin
ABS-09 Collagen-II
ABS-10 Collagen-II
ABS-11 Collagen-II
ABS-12 Collagen-II
GAPDH
Cycles

**ABS-09-PLA+PPF+MgFP; ABS-10-PLA+PDLLA+PPF+MgFP; ABS-11-PLA+Diol+MgFP; ABS-12-PLA+PDLLA+Diol+MgFP; GADPH-Control**

Figure 18

PA Control RUNX2
PA-1 RUNX2
DA Control RUNX2
DA-1 RUNX2
PZ Control RUNX2
PZ-1 RUNX2
DZ Control RUNX2
DZ-1 RUNX2
ABS-09 RUNX2
ABS-10 RUNX2
ABS-11 RUNX2
ABS-12 RUNX2
GAPDH
Cycles

PA Control-PLA+PPF+AgFP; DA Control-PLA+Diol+AgFP; PA-1- PLA+PDLLA+PPF+AgFP; PZ-1- PLA+PDLLA+PPF+ZnFP; PZ Control-PLA+PPF+ZnFP; DZ Control-PLA+Diol+AgFP; DA-1- PLA+PDLLA+Diol+AgFP; DZ-1-PLA+PDLLA+Diol+ZnFP; ABS-09-PLA+PPF+MgFP; ABS-10-PLA+PDLLA+PPF+MgFP; ABS-11-PLA+DIOL+MgFP; ABS-12-PLA+PDLLA+Diol+MgFP; GADPH- Control

**Figure 19**

Chondroitin levels (ng/mL)
ng/mL
14
12
10
8
6
4
2
0
Chondroitin levels-ng/mL
PLA+PPF+AGFP
PLA+Diol+AGFP
PLA+PDLLA+PPF+AGFP
PLA+PDLLA+PPF+ZnFP
PLA+PPF+ZnFP
PLA+Diol+ZnFP
PLA+PDLLA+diol+AGFP
PLA+PDLLA+diol+ZnFP
polymer 9
Polymer 10
Polymer 11
Polymer 12
control
Sample name


Polymer 9-PLA+PPF+MgFP; Polymer 10-PLA+PDLLA+PPF+MgFP; Polymer 11-PLA+Diol+MgFP; Polymer 12-PLA+PDLLA+Diol+MgFP

**Figure 20**

PRE-IMMERSION
POST-IMMERSION
PLA+PDLLA+PPF+AgFP
PLA+PPF+AgFP
PLA+PDLLA+PPF+ZnFP
PLA+PPF+ZnFP
Transmittance (%)
4000 3500 3000 2500 2000 1500 1000 500
Wavenumber (cm-1)

Figure 21

**Sample notation:**

**1-PLA+PPF+ZnFP; 2-PLA+PDLLA+PPF+ZnFP; 3-PLA+PPF+AgFP; 4-PLA+PDLLA+PPF+AgFP**

| Assignments ($cm^{-1}$) | Sample | | | | | | | |
|---|---|---|---|---|---|---|---|---|
| | Pre Immersion | | | | Post Immersion | | | |
| | 1 | 2 | 3 | 4 | 1 | 2 | 3 | 4 |
| OH Stretch (3200-3500) | 3442 (S,B) | 3442 (S,B) | 3182 (S,B) | 3302 (M,B) | 3178 (M,B) | 3277 (W,B) | 3178 (S,B) | 3292 (M,B) |
| CH Stretch (2850-3000) | 2862 (M, Sh) | 2862 (M, Sh) | 2881 (M,Na) | 2995 (M,Na) | 2862 (M, Sh) | - | 2862 (M,Na) | 2939 (M,B) |
| C=O Stretch (1735-1750) | 1747 (M,B) | 1747 (M,B) | 1744 (S,B) | 1744 (S,B) | 1746 (M,B) | 1746 (M,B) | 1746 (S,Sh) | 1744 (S,B) |
| C=C Stretch (1630-1680) | 1626 (S,B) | 1630 (S,B) | 1656 (S,B) | 1665 (S,B) | 1662 (W, Sh) | 1680 (W, Sh) | 1680 (M,Sh) | 1663 (M,Sh) |
| C-H Bend Asymmetric methyl bend (1450-1470) | 1452 (M,B) | 1454 (M,B) | 1455 (W,Sh) | 1454 (M,B) | 1454 (M,B) | 1456 (W,Sh) | 1454 (M,Sh) | 1454 (M,Sh) |
| C-H Bend (1355-1395) | 1384 (M, Sh) | 1384 (M, Sh) | 1364 (M,B) | 1383 (M,B) | 1363 (M,B) | 1381 (M,B) | 1362 (M,B) | 1363 (M,B) |
| $CO_3^{2-}$ (1400-1550) | - | 1507 (W,Sh) | 1541 (W,Sh) | 1541 (W,B) | 1548 (M,Sh) | 1511 (M, Sh) | 1546 (M,Sh) | 1511 (M, Sh) |
| P-O Stretch (1060-1170) | 1098 (M, Sh) | 1094 (M, Sh) | 1086 (M,B) | - | 1079 (M,B) | 1037 (M,B) | 1088 (M,B) | 1088 (M,B) |
| P-O-P Asymmetric Stretch Stretching (850-1050) | 859 (W,B) | - | 957 (W,B) | 864 (W,B) | 912 (W,B) | 916 (W,B) | 1018 (VW,B) | 867 (VW,B) |
| P-O-P Symmetric Stretching (600-800) | 659 (W, Sh) | 657 (W, Sh) | 617 (M,Sh) | 617 (S,Sh) | 688 (W, B) | 690 (W, B) | 654 (M,B) | 687 (M,B) |
| P-O Bend (500-560) | 518 (W,B) | 516 (W,B) | 517 (W,B) | 517 (W,B) | 518 (M, Sh) | 516 (M, Sh) | 516 (M,Sh) | 518 (M,Sh) |

(S-Strong, M-Medium, W-Weak, VW-Very weak; VS-Sharp, Sh-Sharp, B-Broad, Na-Narrow)

Figure 21a

PRE-IMMERSION
POST-IMMERSION
PLA+PDLLA+Diol+AgFP
PLA+Diol+AgFP
PLA+PDLLA+Diol+ZnFP
PLA+Diol+ZnFP
Transmittance (%)
4000 3500 3000 2500 2000 1500 1000 500
Wavenumber (cm-1)

Figure 22

**Sample Notation:**
**1-PLA+Diol+ZnFP; 2-PLA+PDLLA+Diol+ZnFP; 3-PLA+Diol+AgFP;4-PLA+PDLLA+Diol+AgFP**

| Assignments ($cm^{-1}$) | Sample | | | | | | | |
|---|---|---|---|---|---|---|---|---|
| | Pre Immersion | | | | Post Immersion | | | |
| | 1 | 2 | 3 | 4 | 1 | 2 | 3 | 4 |
| OH Stretch (3200-3500) | 3313 (W,B) | 3587 (W,B) | 3502 (W,B) | 3443 (S,B) | 3274 (M,B) | 3586 (W,B) | 3586 (M,B) | 3645 (M,B) |
| CH Stretch (2850-3000) | 2993 (M, Sh) | 2993 (M,Na) | 2943 (M,B) | 2928 (M,Sh) | 2859 (W, Sh) | 2995 (M,Na) | 2931 (M,Sh) | 2876 (M,Na) |
| C=O Stretch (1735-1750) | 1750 (S,B) | 1748 (VS,B) | 1746 (W,B) | 1745 (S,Sh) | 1745 (M,B) | 1747 (S,Sh) | 1746 (S,B) | 1747 (S,Sh) |
| C=C Stretch (1630-1680) | 1664 (VW,Sh) | 1630 (S,B) | - | 1626 (M,B) | 1680 (VW, Sh) | 1680 (W, Sh) | 1604 (W,B) | 1663 (W,Sh) |
| C-H Bend Asymmetric methyl bend (1450-1470) | 1474 (W,B) | 1453 (M,Sh) | - | 1454 (M, Sh) | 1452 (W,B) | 1449 (W,Sh) | 1452 (M,Sh) | 1455 (W, Sh) |
| C-H Bend (1355-1395) | 1375 (M, B) | 1372 (M, B) | 1384 (W,Sh) | 1383 (S,Sh) | 1383 (M,B) | 1373 (M,B) | 1388 (M,B) | 1363 (M,B) |
| $CO_3^{2-}$ (1400-1550) | 1544 (W,B) | 1547 (W, Sh) | 1544 (W,B) | 1509 (W,Sh) | 1511 (M,B) | 1546 (M, Sh) | 1549 (M,Sh) | 1510 (M,Sh) |
| P-O Stretch (1060-1170) | 1174 (M, B) | 1177 (M, B) | 1113 (VS,Sh) | 1195 (M,Sh) | 1198 (W,Sh) | 1175 (M,B) | 1155 (W,B) | 1179 (W,B) |
| P-O-P Asymmetric Stretch stretching (850-1050) | 857 (M,B) | 858 (M,Sh) | 987 (W,B) | 872 (W,B) | - | 858 (M,Sh) | 857 (W,B) | 867 (M,Sh) |
| P-O-P Symmetric Stretching (600-800) | 745 (M, Br) | 747 (M, Sh) | 619 (VS,Sh) | 658 (W,Sh) | 618 (VS,Sh) | 746 (VW, B) | 689 (W,B) | 689 (W,B) |
| P-O Bend (500-560) | 505 (W,B) | 517 (M,Sh) | 518 (W,B) | 597 (M,Sh) | 518 (W, Sh) | 517 (W, Sh) | 518 (M,Sh) | 581 (W,B) |

(S-Strong, M-Medium, W-Weak, VW-Very weak; VS-Very Sharp, Sh-Sharp, M-Medium, B-Broad, Na-Narrow)

Figure 22a

PRE-IMMERSION
POST-IMMERSION
PLA+PDLLA+Diol+MgFP
PLA+Diol+MgFP
PLA+PDLLA+PPF+MgFP
PLA+PPF+MgFP
Transmittance (%)
4000 3500 3000 2500 2000 1500 1000 500
Wavenumber (cm-1)

Figure 23

**Sample notation:**
**1-PLA+PPF+MgFP;2-PLA+PDLLA+PPF+MgFP; 3-PLA+Diol+MgFP;4-PLA+PDLLA+Diol+MgFP**

| Assignments ($cm^{-1}$) | Sample | | | | | | | |
|---|---|---|---|---|---|---|---|---|
| | Pre Immersion | | | | Post Immersion | | | |
| | 1 | 2 | 3 | 4 | 1 | 2 | 3 | 4 |
| OH Stretch (3200-3500) | 3309 (W,B) | 3309 (W,B) | 3588 (W,B) | 3011 (W,B) | 3291 (S,B) | 3291 (W,B) | 3445 (W,B) | 3292 (M,B) |
| CH Stretch (2850-3000) | 2861 (W,Sh) | 2929 (W,Sh) | 2861 (W,Na) | 2861 (VW) | 2823 (M,Sh) | 2971 (M,Sh) | 2879 (M,B) | 2881 (M,B) |
| C=O Stretch (1735-1750) | 1747 (M,B) | 1745 (M,B) | 1747 (S,Sh) | 1746 (M,B) | 1746 (M,V Sh) | 1745 (M,V Sh) | 1746 (S,B) | 1745 (S,Sh) |
| C=C Stretch (1630-1680) | 1626 (W,B) | 1662 (W,B) | 1605 (W,B) | 1662 (W,B) | 1664 (W,Sh) | 1664 (W,Sh) | 1626 (W,Sh) | 16623 (W,Sh) |
| C-H Bend Asymmetric methyl bend (1450-1470) | 1435 (W,Sh) | 1455 (W,B) | 1455 (W,Sh) | 1455 (M,B) | 1454 (W,Sh) | 1453 (W,Sh) | 1454 (W,B) | 1455 (M,Sh) |
| C-H Bend (1355-1395) | 1384 (M,Sh) | 1388 (W,Sh) | 1383 (M,B) | 1380 (W,B) | 1383 (M,Sh) | 1384 (M,Sh) | 1384 (M,B) | 1367 (M,Sh) |
| $CO_3^{2-}$ (1400-1550) | 1546 (W,Sh) | 1546 (W,Sh) | 1546 (M,Sh) | 1547 (W,Sh) | 1548 (M,Sh) | 1550 (M,Sh) | 1550 (W,Sh) | 1550 (W,Sh) |
| P-O Stretch (1060-1170) | 1095 (S,Sh) | 1093 (W,Sh) | 1090 (W,Sh) | 1091 (W,Sh) | 1089 (M,B) | 1090 (M,Sh) | 1097 (M,Sh) | 1087 (M,Sh) |
| P-O-P Asymmetric Stretch stretching (850-1050) | 870 (W,B) | - | 864 (W,B) | - | 867 (W,B) | 854 (W,B) | 867 (W,B) | 867 (M,Sh) |
| P-O-P Symmetric Stretching (600-800) | 618 (M,S) | 657 (W,Sh) | 690 (W,B) | 690 (W,B) | 616 (M,B) | 687 (M,B) | 691 (W,B) | 690 (M,B) |
| P-O Bend (500-560) | 518 (W,Sh) | 518 (W,Sh) | 517 (W,Sh) | 518 (W,Sh) | 518 (M,Sh) | 518 (M,Sh) | 517 (,Sh) | 518 (M,Sh) |

(S-Strong, M-Medium, W-Weak, VW-Very weak; VS-Very Sharp, Sh-Sharp, M-Medium, B-Broad, Na-Narrow)

Figure 23a

PRE-IMMERSION
POST-IMMERSION
PLA+PDLLA+PPF+MgFP
PLA+PDLLA+PPF+AgFP
PLA+PDLLA+PPF+ZnFP
PLA+PDLLA+PPF+MgFP
PLA+PDLLA+PPF+AgFP
PLA+PDLLA+PPF+ZnFP
Transmittance (%)
4000 3500 3000 2500 2000 1500 1000 500
Wavenumber (cm-1)
4000 3500 3000 2500 2000 1500 1000 500
Wavenumber (cm-1)

Figure 24

**Sample Notation:**
**1-PLA+PDLLA+PPF+ZnFP; 2-PLA+PDLLA+PPF+AgFP; 3-PLA+PDLLA+PPF+MgFP**

| Assignments ($cm^{-1}$) | Sample | | | | | |
|---|---|---|---|---|---|---|
| | Pre Immersion | | | Post Immersion | | |
| | 1 | 2 | 3 | 1 | 2 | 3 |
| OH Stretch (3200-3500) | 3613 (VW,B) | 3503 (VW,B) | 3612 (VW,B) | 3645 (M,B) | 3642 (M,B) | 3436 (S,B) |
| CH Stretch (2850-3000) | 2861 (VW,B) | 2880 (VW,B) | 2880 (W,B) | 2881 (M,B) | 2878 (M,B) | 2860 (W,B) |
| C=O Stretch (1735-1750) | 1746 (M,B) | 1748 (M,B) | 1746 (M,Sh) | 1745 (VS,Sh) | 1745 (VS,Sh) | 1765 (VW,B) |
| C=C Stretch (1630-1680) | 1662 (VW,B) | 1624 (VW,B) | 1662 (W,B) | 1663 (W,Sh) | 1665 (W,Sh) | 1630 (M,B) |
| C-H Bend Asymmetric methyl bend (1450-1470) | 1455 (M,B) | 1455 (M,Sh) | 1454 (W,Sh) | 1455 (M,Sh) | 1451 (M,Sh) | 1447 (W,B) |
| C-H Bend (1355-1395) | 1380 (W,B) | 1385 (M,B) | - | 1367 (M,Sh) | 1383 (M,Sh) | 1316 (M,Na) |
| $CO_3^{2-}$ (1400-1550) | 1547 (W,Sh) | 1547 (W,Sh) | 1511 (M,Sh) | 1550 (W,Sh) | 1589 (W,Sh) | 1536 (W,Sh) |
| P-O Stretch (1060-1170) | 1091 (W,Sh) | 1094 (M,Sh) | 1083 (W,Na) | 1087 (M,Sh) | 1090 (M,Sh) | 1093 (S,Na) |
| P-O-P Asymmetric Stretch stretching (850-1050) | - | 870 (W,B) | - | 867 (M,Sh) | 867 (M,Sh) | 871 (VW,B) |
| P-O-P Symmetric Stretching (600-800) | 690 (W,B) | 691 (VW,B) | 690 (W,B) | 690 (M,Sh) | 688 (M,B) | 659 (M,Sh) |
| P-O Bend (500-560) | 518 (W,Sh) | 518 (W,Sh) | 518 (W,Sh) | 518 (M,Sh) | 517 (M,Sh) | 597 (S,Sh) |

(S-Strong, M-Medium, W-Weak, VW-Very weak; VS-Very Sharp, Sh-Sharp, M-Medium, B-Broad, Na-Narrow)

**Figure 24a**

Post immersion
Pre immersion
Transmittance (%)
4000
3500
3000
2500
2000
1500
1000
500
Wavenumber (cm-1)

Figure 25

| STRIP | Assignments ($cm^{-1}$) | | | | | | | | | | |
|---|---|---|---|---|---|---|---|---|---|---|---|
| | OH Stretch (3200-3500) | CH Stretch (2850-3000) | C=O Stretch (1735-1750) | C=C Stretch (1630-1680) | C-H Bend Asymmetric methyl bend (1450-1470) | C-H Bend (1355-1395) | $CO_3^{2-}$ (1400-1550) | P-O Stretch (1060-1170) | P-O-P Asymmetric Stretch stretching (850-1050) | P-O-P Symmetric Stretching (600-800) | P-O Bend (500-560) |
| PRE IMMERSION | 3507 (VW,B) | 2877 (VW,B) | 1754 (M,B) | 1662 (VW,B) | 1456 (M,B) | 1384 (W,B) | 1547 (W,Sh) | 1093 (M,Sh) | 871 (W,B) | 682 (VW,B) | 518 (W,Sh) |
| POST IMMERSION | 3642 (M,B) | 2878 (M,B) | 1743 (VS,Sh) | 1664 (W,Sh) | 1455 (M,Sh) | 1385 (W,B) | 1586 (M,Sh) | 1099 (M,Sh) | 867 (M,Sh) | 688 (M,B) | 517 (M,Sh) |

(S-Strong, M-Medium, W-Weak, VW-Very weak; VS-Very Sharp, Sh-Sharp, M-Medium, B-Broad, Na-Narrow)

**Figure 25a**

Post immersion
Pre immersion
Transmittance (%)
4000
3500
3000
2500
2000
1500
1000
500
Wavenumber ($cm^{-1}$)

Figure 26

| CYLINDER | Assigments ($cm^{-1}$) | | | | | | | | | | |
|---|---|---|---|---|---|---|---|---|---|---|---|
| | OH Stretch (3200-3500) | CH Stretch (2850-3000) | C=O Stretch (1735-1750) | C=C Stretch (1630-1680) | C-H Bend Asymmetric methyl bend (1450-1470) | C-H Bend (1355-1395) | $CO_3^{2-}$ (1400-1550) | P-O Stretch (1060-1170) | P-O-P Asymmetric Stretch stretching (850-1050) | P-O-P Symmetric Stretching (600-800) | P-O Bend (500-560) |
| PRE IMMERSION | 3566 (M,B) | 2876 (S. Na) | 1746 (M,Sh) | 1643 (W,Sh) | 1452 (M,Sh) | 1339 (W,Sh) | 1510 (M,SH) | 1076 (M,B) | 891 (VW,B) | 634 (M,Na) | 518 (W,Sh) |
| POST IMMERSION | 3262 (W,B) | 2862 (M,Na) | 1751 (M,B) | - | 1449 (W,B) | 1384 (W,Sh) | 1545 (M,B) | 1139 (S,B) | 920 (M,B) | 664 (VW,B) | 564 (M,B) |

(S-Strong, M-Medium, W-Weak, VW-Very weak; VS-Very Sharp, Sh-Sharp, M-Medium, B-Broad, Na-Narrow)

**Figure 26a**

Post immersion
Pre immersion
Post immersion
Pre immersion

Post immersion and Pre immersion photographs of the strip and the cylinder

Figure 27

Pre immersion at 20μm
Pre immersion at 5μm
Post immersion at 20μm
Post immersion at 5μm

Figure 28

L1 = 654.31 µm

**Figure 28a**

Figure 29

Figure 29a

Pre immersion at 5000µm

Pre immersion at 50µm

Figure 30

**Post immersion at 20µm** **Post immersion at 10µm**

Figure 30a

Energy (KeV)
Pre immersion
Energy (KeV)
Post immersion

Figure 30b

Pre immersion at 50μm

Post immersion at 50μm

Figure 31

Figure 32

Day 0
Day 1
Day 9
Day 16

Figure 33

Figure 34

Line of Section
cortical bone
cortical bone
Area seen in Section (HPE)

Figure 35

Yellow arrow- Cartilage
Green Arrow- Composite material
Red arrow- Woven bone

VON KOSSA STAIN

Thick black arrow-Cartilage
Thick red arrow-Osteoid
Thin long balck arrow- Composite material
Thin long red arrow- Woven bone
Red box- RIM of osteoblastic cells

HEMATOXYLIN AND EOSIN STAIN

Figure 36 A&B

Mature lamellar bone in continuity with the composites
Corticotomy by the surgeon
Another fragment of fracture femur
Woven bone

Modified Tetrachrome stain

**Figure 37A**

Cartilage getting ossified
Woven bone

Modified Tetrachrome stain

**Figure 37b**

Abundant osteoblasts and newly laid osteoid

Modified Tetrachrome stain

**Figure 37c**

Neovascularization
Fibroblasts

Modified Tetrachrome Stain

**Figure 37d**

Figure 38

Day 0
Day 1
Day 14
Day 19

Figure 39a

Day 0
Day 1
Day 14
Day 19

Figure 39b

Day 0
Day 3
Day 14
Day 19

**Figure 39 c**

Figure 40 a

Figure 40 b

Figure 40c

Specimen-A
Specimen-B
Specimen-C

Figure 41

A
B
C

Figure 42 a

A
B
C

Figure 42b

Fusion of both layers of composite
Woven bone at the cortical defect created
Cortical Bone of the Femur
Fibrous infiltration of the superficial layer of glass

**Figure 43a**

Abundance of Neo Vascularisation of the superficial layer of composite at higher magnification.

**Figure 43b**

Fibrous infiltration of the glass
composite

**Figure 43c**

Neo vascularisation in higher magnification

Freshly infiltrated young fibroblasts

**Figure 43 d**

Woven bone formed to fill the corticotomy
Fused layer of the composite
Bioconversion of the composite

**Figure 44a**

Woven bone
Osteoid binding between the composite and bone
Residual composite

**Figure 44b**

Fragmented scaffold getting converted into woven bone
Osteoid promoting adherence of the scaffold to the newbone formation
Bioconversion of the composite

Figure 44c

Woven bone formed from the fragmented scaffold
Undissolved composite segment
Osteoid formation inside the scaffold

Figure 44d

Machine Controller
Insulated Box
Extruder cooling Temperature controller
Bone Graft Machine
Chamber Hot Temperature controller
Power Button on

**Figure 45 (a)**

Linear slurry Pressure system
Slurry Flow System
Slurry Cooling System
Heat Built Plate
CAUTION

**Figure 45(b)**

Printed Output

@ Print

Figure 45(c)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4888413 A
- IN 5760CHE2013
- IN 5990CHE2013
- IN 5989CHE2013